# EUROPEAN PATENT APPLICATION

(11) **EP 4 378 925 A1**
(43) Date of publication of application: **05.06.2024**
(21) Application number: 21951294.4
(22) Date of filing: 29.07.2021
(51) Int. Cl.: C07C 269/00, C07C 269/06, C07C 271/22, C07C 231/12, C07C 235/74, C07D 233/56, C07D 207/46, C07C 51/41, C07C 53/18, C07C 201/12, C07C 205/43, C07C 41/16, C07C 43/178, C07K 14/61, A61K 38/27, A61K 47/60, A61K 47/68, A61P 5/06, A61P 35/00

(54) **UNNATURAL AMINO ACID AND APPLICATION THEREOF, RECOMBINANT PROTEIN CONTAINING SAME, AND RECOMBINANT PROTEIN CONJUGATE**

(71) Applicant: Novocodex Biopharmaceuticals Co., Ltd., Shaoxing, Zhejiang 312366 (CN)
(72) Inventor: YANG, Jinwei, Shaoxing, Zhejiang 312366 (CN); YE, Chenghao, Shaoxing, Zhejiang 312366 (CN); XIA, Gang, Shaoxing, Zhejiang 312366 (CN); HUO, Pengchao, Shaoxing, Zhejiang 312366 (CN); DING, Wen, Shaoxing, Zhejiang 312366 (CN); CHEN, Longfei, Shaoxing, Zhejiang 312366 (CN); JIAO, Lin, Shaoxing, Zhejiang 312366 (CN); HUANG, Hao, Shaoxing, Zhejiang 312366 (CN); HENG, Xin, Shaoxing, Zhejiang 312366 (CN); ZHU, Jingjing, Shaoxing, Zhejiang 312366 (CN); YING, Yuebin, Shaoxing, Zhejiang 312366 (CN); LIANG, Xuejun, Shaoxing, Zhejiang 312366 (CN)
(74) Representative: dompatent von Kreisler Selting Werner - Partnerschaft von Patent- und Rechtsanwälten mbB
(86) International application number: PCT/CN2021/109277
(87) International publication number: WO 2023/004687

(57) **Abstract**

The invention provides an unnatural amino acid, which is a compound with a structure as shown in formula (I) or an enantiomer thereof. The invention further provides use of the unnatural amino acid. Furthermore, the invention further provides a recombinant protein containing the unnatural amino acid and a protein conjugate prepared from the recombinant protein. The unnatural amino acid provided by the invention is simple and convenient to prepare, safe in nature, not easy to be inactivated when being inserted into a protein, and has a high binding rate with a coupling moiety, and the obtained conjugate is higher in stability. The unnatural amino acid provided by the invention is applicable to numerous fields, especially in preparation of a recombinant protein or a recombinant protein conjugate,

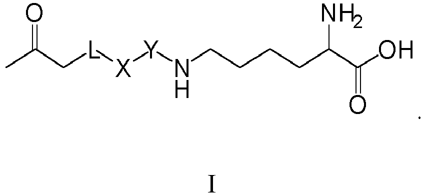

## Description

### TECHNICAL FIELD

The present invention relates to the field of biopharmaceuticals, and more particularly, to an unnatural amino acid, a recombinant protein containing such unnatural amino acid and a conjugate formed with the recombinant protein.

### BACKGROUND

The development of various scientific research and product applications can be realized by introducing unnatural amino acids containing special groups into proteins. For example, the introduction of photosensitive unnatural amino acids into the proteins or the special labeling to the unnatural amino acids is convenient for studying the interaction between the proteins. Another example is the unnatural amino acids which are used for the directional evolution of enzymes to improve the activity and stability of the enzymes, or to facilitate the efficient immobilization of the enzymes. For another example, safe live bacterium or live virus vaccines may be prepared by using the characteristic that the insertion of the unnatural amino acids which cannot be realized in conventional hosts. An important application of using codon expansion technology in introducing the unnatural amino acids into the proteins lies in the site-specific modification of the proteins, which could improve the function, stability and half-life of the proteins, and may be used for the development of innovative biological drugs. At present, a series of achievements have been made, such as the preparation of long-acting recombinant proteins from PEG site-specific coupled recombinant human growth hormones, the development of antibody-drug conjugates from small molecular toxin site-specific coupled monoclonal antibodies, and the like. Thus, it can be seen that the unnatural amino acids have a very important role and a very wide range of applications.

The prior arts (such as CN 102838671B, CN 106146663A, and J. Am. Chem. Soc. 2009, 131, 8720) disclose an unnatural amino acid Lys-azido, with a structural formula as follows:

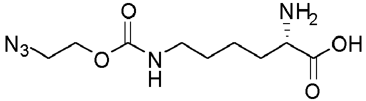

An azide structure (-N₃) at the end of the Lys-azido can be chemically linked with a carrier drug (such as PEG) modified with an alkyne-containing structure (such as BCN, i.e. 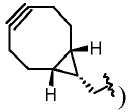 to obtain a conjugate (for example, Chinese patent CN 103153927B), which has very high specific selectivity. However, alkyne structures with high costs need to be introduced in this coupling method and chemical modification method, and an acceptable drug-antibody coupling ratio can be obtained only when the alkyne structures are used in larger equivalent, which increases corresponding production costs, and has a complicated technological process and harsh technological conditions.

Therefore, it is necessary to develop an unnatural amino acid with a novel structure, simple preparation and a low cost, so as to expand the types and applications of amino acids.

### SUMMARY

In order to overcome the defects in the prior art, one object of the present invention is to provide an unnatural amino acid.

Another object of the present invention is to provide a use of the unnatural amino acid.

Yet another object of the present invention is to provide a recombinant protein and a recombinant protein conjugate.

The unnatural amino acid provided by the present invention is a compound with a structure as shown in formula (I) or an enantiomer thereof, 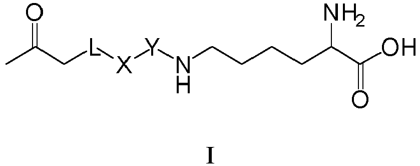
wherein X represents -O-, -S-, -NH- or -CH₂-, Y represents -O-, -S-, -C(O)-, -S(O)- or -CH₂-, and L represents substituted or unsubstituted C0-C20 linear or branched alkylene, wherein one or more -CH₂- are optionally substituted by one or more of -O-, -S-, -NH-, -C(O)- and -S(O)-;
when X represents -O- and Y represents -C(O)-, L does not represent -CH₂CH₂-; and
when L represents a substituent, the substituent is selected from one or more of hydroxyl, sulfhydryl, halogen, nitro, cyano, alkyl, alkenyl, alkynyl, alkoxy, acyl, acylamino, carboxyl, ester, amino, sulfonyl, sulfinyl, cycloalkyl, heterocyclyl, aryl and heteroaryl.

As shown in Example 7, the inventor of the present invention found that, besides the high cost and the complicated process, the azide structure (-N₃) at the end of Lys-azido was easily reduced into an amino structure (-NH₂) when the azide structure was inserted into a protein, thus losing coupling activity, so that the reduction reaction reduces a yield in a technology preparation process.

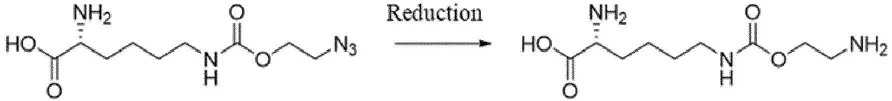

Carbonyl is introduced at the end of the unnatural amino acid provided by the present invention as an active reaction group, so that the unnatural amino acid is not only novel in structure, simple and convenient to prepare, but also mild in coupling conditions and low in production cost, and is not easy to lose reaction activity due to a structural change when being inserted into a protein sequence. In addition, the unnatural amino acid provided by the present invention further contains alkylene with a certain chain length, so that the compound has better flexibility and is easier to form various conjugates.

In the unnatural amino acid provided by the present invention, "C0-Cn" comprises C0-C1, C0-C2, ..., C0-Cn, and C0 indicates that the group does not exist, and C atoms at two ends of the group are directly connected to form a bond. For example, the "C0-C6" group refers to that there are 0 to 6 carbon atoms in this part, which means that the group does not exist, the group contains 1 carbon atom, 2 carbon atoms, 3 carbon atoms, 4 carbon atoms, 5 carbon atoms or 6 carbon atoms. The "C6-C10" group refers to that there are 6 to 10 carbon atoms in this part, which means that the group contains 6 carbon atoms, 7 carbon atoms, 8 carbon atoms, 9 carbon atoms or 10 carbon atoms.

In some preferred embodiments of the present invention, the substituent may be selected from one or more of hydroxyl, sulfhydryl, halogen, nitro, cyano, C1-C6 alkyl, C1-C6 alkoxy, acyl, acylamino, carboxyl, ester, amino, sulfonyl, sulfinyl, C3-C8 cycloalkyl, C3-C8 heterocyclyl, C6-C20 aryl and C4-C10 heteroaryl.

In some preferred embodiments of the present invention, L may represent C0-C10 linear or branched alkylene, and one or more -CH₂- are optionally substituted by -O-. In some more preferred embodiments of the present invention, , L represents C0-C6 linear or branched alkylene, and one or more -CH₂- are optionally substituted by -O-.

In some preferred embodiments of the present invention, X may represent -O-, -S-, -NH- or -CH₂-.

In some preferred embodiments of the present invention, Y may represent -C(O)-.

In some preferred embodiments of the present invention, the unnatural amino acid is a compound with a structure as shown in formula (I-1), 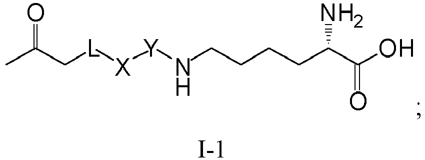 wherein X, Y and L are each independently as defined in any one of the above technical solutions.

In some preferred embodiments of the present invention, the unnatural amino acid is a compound with a structure as shown in formula (I-2), formula (I-3), formula (I-4) or formula (I-5), 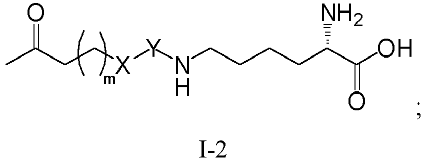 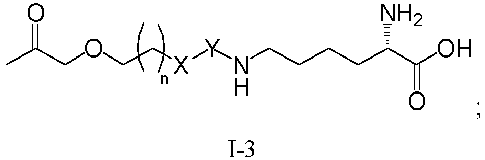 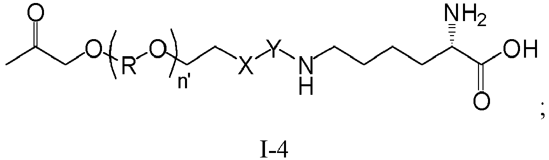 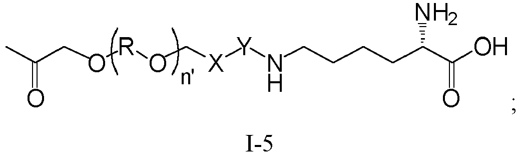
wherein X and Y are each independently as defined in any one of the above technical solutions, m and n each independently represents an integer of 0 to 8 (for example, 0, 1, 2, 3, 4, 5, 6, 7 or 8), preferably represents an integer of 0 to 5, and more preferably represents 0, 1, 2 or 3;
when X represents -O- and Y represents -C(O)-, m does not represent 1; and
R represents C1-C4 linear or branched alkylene, and preferably represents -CH₂-, -CH₂CH₂- or -CH₂CH₂CH₂-; and n' represents an integer of 0 to 5 (for example, 0, 1, 2, 3, 4 or 5), preferably represents an integer of 0 to 3, and more preferably represents 0, 1 or 2.

The unnatural amino acid provided by the present invention comprises optically pure enantiomer and racemate.

In some more preferred embodiments of the present invention, the unnatural amino acid provided by the present invention is a compound with one of the structures as follows:

| | |
|---|---|
| (1) NOBK | 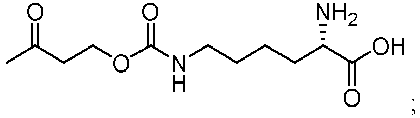 |
| (2) NOHK | 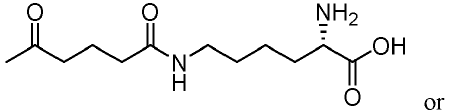 |
| (3) NOPK | 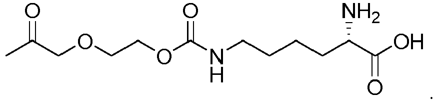 |

The present invention further provides use of the unnatural amino acid according to any one of the above technical solutions in preparation of a recombinant protein or a recombinant protein conjugate.

In the use of the present invention, the recombinant protein may be a recombinant protein obtained by inserting the unnatural amino acid according to any one of the above technical solutions into any kind of protein common in the art at any site in any quantity. The recombinant protein conjugate may be a conjugate obtained by coupling any recombinant protein obtained with a coupling moiety common in the art, wherein the coupling moiety may include, but is not limited to, one or more of polymers (such as polyethylene glycol with any molecular weight), proteins, polypeptides or small molecule drugs.

In some preferred embodiments of the present invention, the recombinant protein may be a recombinant human growth hormone, and the recombinant protein conjugate may be a recombinant human growth hormone-polyethylene glycol conjugate.

The present invention further provides a recombinant protein, wherein at least one site in the amino acid sequence of the recombinant protein is the unnatural amino acid according to any one of the above technical solutions.

Further, the recombinant protein may have a structure as shown in formula (II), 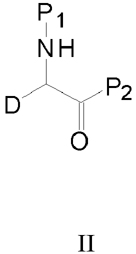 in the formula (II), D represents a residue of the unnatural amino acid according to any one of the above technical solutions with an aminocarboxylic acid part removed, and P₁ and P₂ respectively represent connecting parts of the amino and the carboxyl of the unnatural amino acid in the amino acid sequence.

The recombinant protein provided by the present invention may be prepared by a preparation method common in the art, including realizing cloning and expression of the recombinant protein containing the unnatural amino acid by a gene codon expansion technology.

The recombinant protein provided by the present invention may be a recombinant protein obtained by inserting the unnatural amino acid according to any one of the above technical solutions into any kind of protein common in the art at any site in any quantity, such as a recombinant human growth hormone.

The present invention further provides a recombinant protein conjugate, wherein the recombinant protein conjugate is formed by forming an oxime bond with a terminal carbonyl of the unnatural amino acid in the recombinant protein according to any one of the above technical solutions and a coupling moiety containing an "NH₂-O-" terminal group. Further, the recombinant protein conjugate may have a structure as shown in formula (III), 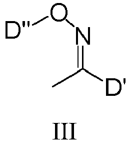 in the formula (III), D' represents a residue of the recombinant protein according to any one of the above technical solutions with the terminal carbonyl part of the unnatural amino acid removed, and D" represents a residue of the coupling moiety with the "NH₂-O-" terminal group removed.

In the recombinant protein conjugate provided by the present invention, the coupling moiety may include one or more of polymers (such as polyethylene glycol with any molecular weight), proteins, polypeptides or small molecule drugs. Different kinds of coupling moietys may be separately coupled with the recombinant protein, or different kinds of coupling moietys may be connected first and then coupled with the recombinant protein.

In some preferred embodiments of the present invention, the recombinant protein may be a recombinant human growth hormone, and the coupling moiety containing the "NH₂-O-" terminal group may be polyethylene glycol containing an "NH₂-O-" terminal group.

In some more preferred embodiments of the present invention, the polyethylene glycol containing the "NH₂-O-" terminal group has a structural formula as follows: 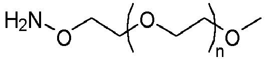 wherein the molecular weight of the polyethylene glycol containing the "NH₂-O-" terminal group may be 10 KD to 100 KD, including, but being not limited to, molecular weight values of about 10 KD, 20 KD, 30 KD, 40 KD, 50 KD, 60 KD, 70 KD, 80 KD, 90 KD and 100 KD, or a molecular weight range of any combination. Preferably, the molecular weight of the polyethylene glycol containing the "NH₂-O-" terminal group may be 20 KD to 50 KD.

In some more preferred embodiments of the present invention, the amino acid sequence of the recombinant human growth hormone is as shown in SEQ ID NO: 1; and further preferably, a corresponding 107^{th} site in the amino acid sequence of SEQ ID NO: 1 is the unnatural amino acid according to any one of the above technical solutions.

When the recombinant protein is the recombinant human growth hormone, the present invention further provides use of the recombinant protein conjugate according to any one of the above technical solutions in preparation of a drug for treating growth and development disorders caused by endogenous growth hormone secretion insufficiency, growth and development disorders caused by Turner syndrome or adult growth hormone deficiency.

The technical solutions provided by the present invention have the following advantages.
(1) The terminal carbonyl is introduced into the structure of the unnatural amino acid provided by the present invention, and compared with the current terminal azido unnatural amino acid (such as Lys-azido), the unnatural amino acid is simple and convenient to prepare, good in safety, not easy to inactivate when being inserted into a protein, and high in binding rate with a coupling moiety, and the obtained conjugate is better in stability.
(2) As an amino acid derivative, the unnatural amino acid provided by the present invention may have the properties of amino acid, thus expanding potential types of amino acids, so that the unnatural amino acid may be applied to numerous fields as the amino acid derivative, especially in preparation of a recombinant protein or a recombinant protein conjugate.
(3) The unnatural amino acid provided by the present invention may be successfully recognized in a prokaryotic expression system and an eukaryotic expression system and inserted into a protein, thus producing a protein containing the unnatural amino acid at a specific site, so that recombinant proteins with different physical and chemical properties and biochemical activities may be formed, and types and potential application scopes of the proteins are expanded. In addition, the unnatural amino acid of the present invention has higher expression efficiency in protein and better practicability.
(4) Because the recombinant protein provided by the present invention contains the unnatural amino acid of the present invention, the terminal carbonyl active group contained in the unnatural amino acid may easily form a protein conjugate (or conjugate), such as a polyethylene glycol conjugate and a polyethylene glycol-active drug conjugate. These protein conjugates may have many improved biological activities (such as anti-tumor activity) due to property modification.
(5) The present invention further provides a novel protein conjugate platform, and multiple types of proteins and multiple types of coupling moietys may be bound through the novel unnatural amino acid contained in protein and the coupling moiety connected.

### DESCRIPTION OF THE DRAWINGS

FIG. 1 is a profile of expression plasmid pET21-rhGH107 for a recombinant human growth hormone in Example 4.

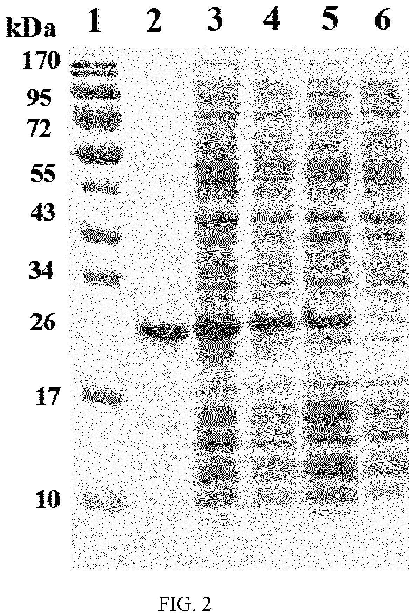

FIG. 2 is an SDS-PAGE electrophoretogram of fermentation products obtained by adding different types of unnatural amino acids in Example 4, wherein various lanes respectively represent the followings: Lane 1, protein molecular weight Marker; Lane 2, wild-type recombinant human growth hormone; Lane 3, expression product of recombinant human growth hormone added with NOBK; Lane 4, expression product of recombinant human growth hormone added with NOHK; Lane 5, expression product of recombinant human growth hormone added with NOPK; and Lane 6, expression product of recombinant human growth hormone not added with any unnatural amino acid.
FIG. 3 is an SDS-PAGE electrophoretogram of coupling products of recombinant human growth hormone containing an unnatural amino acid coupled with PEG in Example 4, wherein various lanes respectively represent the followings: Lane 1, molecular weight Marker; Lane 2, wild-type recombinant human growth hormone; Lane 3, coupling product of recombinant human growth hormone containing NOBK coupled with PEG; Lane 4, coupling product of recombinant human growth hormone containing NOHK coupled with PEG; and Lane 5, coupling product of recombinant human growth hormone containing NOPK coupled with PEG.
FIG. 4 is an SDS-PAGE electrophoretogram of purified PEG-coupled recombinant human growth hormones in Example 4, wherein various lanes respectively represent the followings: Lane 1, molecular weight Marker; Lane 2, wild-type recombinant human growth hormone; and Lane 3, coupling product of recombinant human growth hormone containing NOPK coupled with PEG.
FIG. 5A to FIG. 5E are cell activity curves in Example 4, wherein FIG. 5A is a cell activity curve of rhGH standard product of National Institutes for Food and Drug Control; FIG. 5B is a cell activity curve of Jintropin^{®}; FIG. 5C is a cell activity curve of Polyethylene Glycol Recombinant Human Somatropin Injection^{®}; FIG. 5D is a cell activity curve of rhGH (NOPK); and FIG. 5E is a cell activity curve of PEG-rhGH (NOPK).
FIG. 6 is a fluorescence micrograph of CHO cells transiently expressing inserted unnatural amino acids in Example 5.
FIG. 7 is a profile of an expression plasmid pCDNA3.1-Trastuzumab-UAG142 in Example 6.
FIG. 8A to FIG. 8C are respectively HIC-HPLC spectra of trastuzumab containing NOPK, trastuzumab containing NOHK and trastuzumab containing NOBK coupled with a toxin in Example 6.
FIG. 9 is an inhibitory effect graph of the trastuzumab containing NOPK and DM1-modified trastuzumab containing NOPK on BT-474 cells in Example 6.
FIG. 10A and FIG. 10B are respectively mass spectra of rhGH with a mutation into Lys-azido at 140^{th} site and rhGH with a mutation into NOPK at 140^{th} site in Example 7.

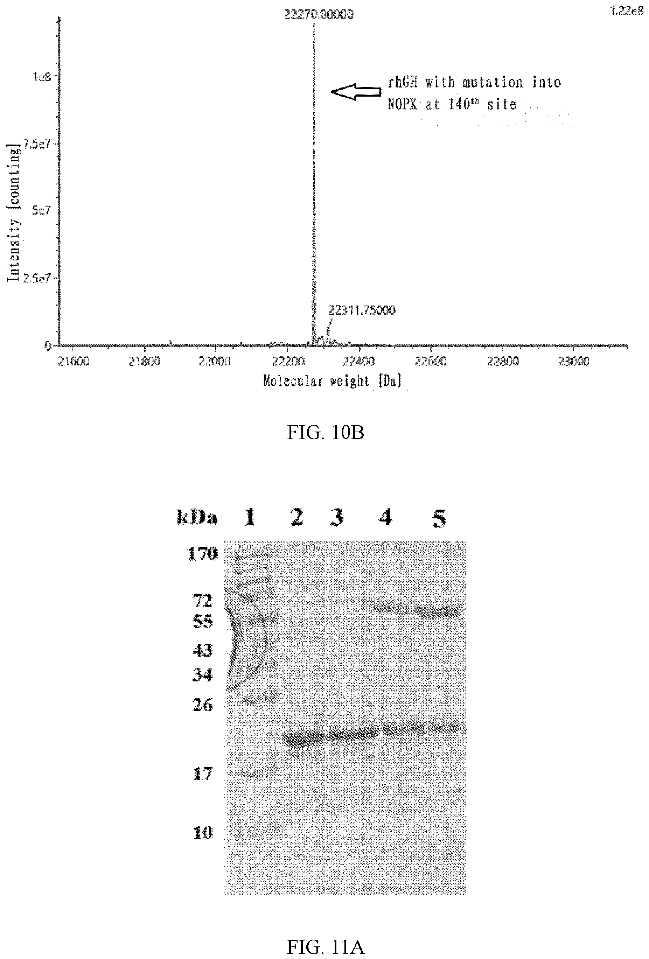

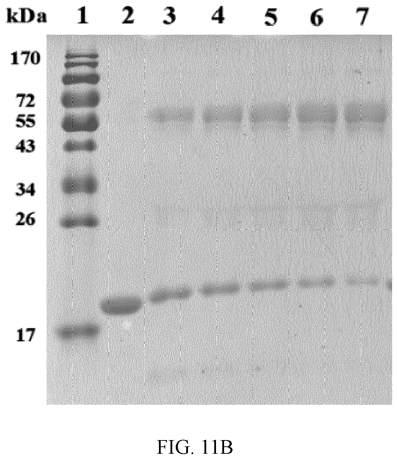

FIG. 11A and FIG. 11B are respectively SDS-PAGE electrophoregrams of coupling processes of rhGH with a mutation into Lys-azido at 140^{th} site and rhGH with a mutation into NOPK at 140^{th} site in Example 7 with 30KD PEG, wherein various lanes in FIG. 11A respectively represent the followings: Lane 1, molecular weight Marker; Lane 2, wild-type recombinant human growth hormone; Lane 3, rhGH-Lys-azido-140; Lane 4 , product of coupling reaction for 72 hours when rhGH-Lys-azido-140: 30K BCN-PEG is 1:15 (molar ratio, the same below); and Lane 5, product of coupling reaction for 72 hours when rhGH-Lys-azido-140: 30K BCN-PEG is 1:25. Various lanes in FIG. 11B respectively represent the followings: Lane 1, molecular weight Marker; Lane 2, rhGH-NOPK-140; Lane 3, product of coupling reaction for 6 hours when rhGH-NOPK-140: 30K BCN-PEG is 1:15; Lane 4, product of coupling reaction for 9 hours when rhGH-NOPK-140: 30K BCN-PEG is 1:15; Lane 5, product of coupling reaction for 12 hours when rhGH-NOPK-140: 30K BCN-PEG is 1:15; Lane 6, product of coupling reaction for 24 hours when rhGH-NOPK-140: 30K BCN-PEG is 1:15; and Lane 7, product of coupling reaction for 48 hours when rhGH-NOPK-140: 30K BCN-PEG is 1:15.

### EMBODIMENT

The technical solutions of the present invention will be further described in detail hereinafter with reference to the specific examples.

Unless otherwise specified, the reagents or raw materials used in the examples of the present invention are all commercially available.

### Example 1 Preparation of unnatural amino acid NOBK

The structural formula of NOBK was as follows:

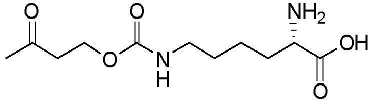

The reaction process was shown in the following figure:

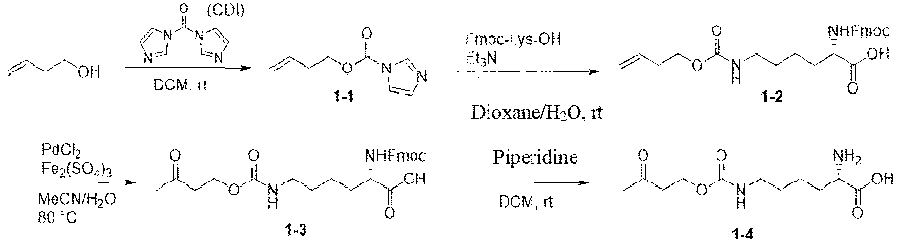

The preparation process included the following steps.
a) 3-butene-1-ol (2.00 g, 27.74 mmol) and a solvent DCM (15.0 mL) were added into a reaction flask, and then CDI (4.50 g, 27.74 mmol) was added at 0°C. The mixture was reacted at room temperature for 18 hours, and then added with 10 mL of water, and extracted with DCM for 3 times. Organic phases were combined, dried with anhydrous sodium sulfate, filtered, and concentrated under a reduced pressure to obtain a product **1-1** (4.50 g, yield 98%).
b) The product **1-1** (0.55 g, 3.31 mmol) and Fmoc-lysine hydrochloride (1.12 g, 2.76 mmol) were added into a reaction flask, added with 9 mL of dioxane and 3 mL of water (v/v=3:1), and then added with triethylamine (0.70 g, 6.9 mmol). The mixture was reacted at room temperature for 24 hours, added with 20 mL of 1 M HCl solution to adjust the pH value to be about 2, and extracted with ethyl acetate for 3 times. Organic phases were combined, dried with anhydrous sodium sulfate, filtered, concentrated under a reduced pressure, and purified by column chromatography (eluent: DCM: MeOH = 30:1) to obtain a product **1-2** (0.17 g, yield 14%).
c) The product **1-2** (0.13 g, 0.28 mmol), ferric sulfate (0.17 g, 0.42 mmol) and palladium chloride (15 mg, 0.03 mmol) were added into a two-neck reaction flask, added with 3 mL of acetonitrile and 0.5 mL of water. After the mixture was refluxed at 60°C for 24 hours, the mixture was cooled to room temperature, and extracted with DCM for 3 times. Organic phases were combined, concentrated under a reduced pressure, and purified by column chromatography (eluent: DCM: MeOH = 20:1) to obtain a product **1-3** (91.0 mg, yield 68%).
d) The product **1-3** (91.0 mg, 0.19 mmol) was dissolved in DCM (2 mL) in a reaction flask, added with piperidine (36.4 mg, 0.43 mmol), reacted at room temperature for 3 hours, concentrated under a reduced pressure, and purified by column chromatography (eluent: MeOH: H₂O = 40:10:1) to obtain the target product **NOBK 1-4** (20.1 mg, yield 41%).

¹H NMR (400 MHz, heavy water) δ 4.20 (t, *J* = 6.8 Hz, 2H), 3.64 (t, *J* = 6.0 Hz, 1H), 3.02 (t, *J* = 6.0 Hz, 2H), 2.16 (s, 3H), 2.07 - 1.80 (m, 4H), 1.69 - 1.52 (m, 2H), 1.52 - 1.36 (m, 2H).

### Example 2 Preparation of unnatural amino acid NOHK

The structural formula of NOHK was as follows:

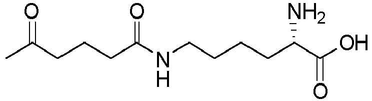

The reaction process was shown in the following figure:

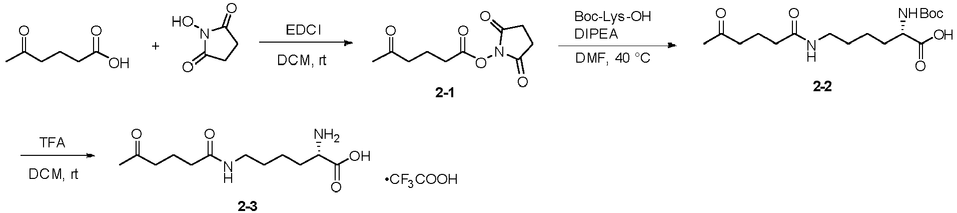

The preparation process included the following steps.
a) 5-oxohexanoic acid (2.60 g, 20.0 mmol), N-hydroxysuccinimide (2.30 g, 20.0 mmol) and EDCI (3.83 g, 20.0 mmol) were added into a reaction flask, and then a solvent DCM (100 mL) was added. The mixture was reacted at room temperature for 18 hours, washed with water for 3 times, dried with anhydrous sodium sulfate, and concentrated under a reduced pressure to obtain a product **2-1** (4.62 g, yield 102%), which was directly used in next step without purification.
b) The product **2-1** (4.62 g, 20.35 mmol) and Boc-lysine (4.42 g, 16.96 mmol) were added into a reaction flask, and then added with DIPEA (4.07 mL, 42.4 mmol) and a solvent DMF (50 mL). The mixture was reacted at 40°C for 24 hours, washed with water for 5 times, dried with anhydrous sodium sulfate, filtered, concentrated under a reduced pressure, and purified by column chromatography (eluent: DCM: MeOH = 30:1) to obtain a product **2-2** (4.36 g, yield 60%).
c) The product **2-2** (4.36 g), a solvent DCM (10 mL) and an equivalent amount (10 mL) of trifluoroacetic acid (TFA) were added into a reaction flask. The mixture was stirred at room temperature for 20 minutes, concentrated under a reduced pressure, then added with an acetonitrile solvent for multiple times to remove excess trifluoroacetic acid under reduced pressure concentration to obtain the target product **2-3** (4.10 g, yield 91%).

¹H NMR (400 MHz, heavy water) δ 3.75 (t, *J* = 6.0 Hz, 1H), 3.21 (t, *J* = 6.8 Hz, 2H), 2.61 (t, *J* = 7.2 Hz, 2H), 2.26 (d, *J* = 7.4 Hz, 2H), 2.23 (s, 3H), 1.96 - 1.79 (m, 4H), 1.63 - 1.52 (m, 2H), 1.49 - 1.34 (m, 2H).

### Example 3 Preparation of unnatural amino acid NOPK

The structural formula of NOPK was as follows:

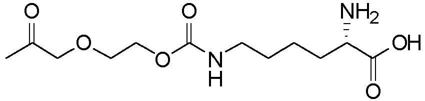

The reaction process was shown in the following figure:

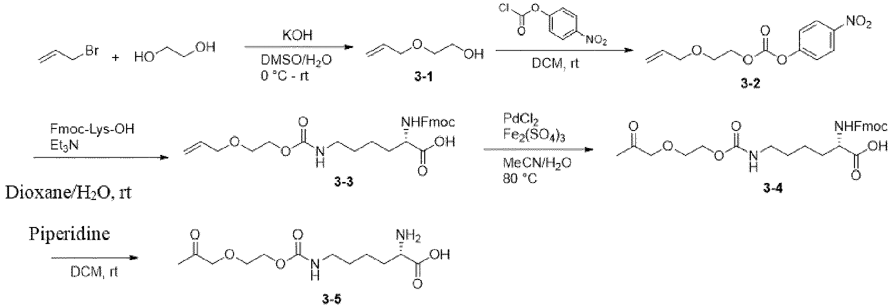

The preparation process included the following steps.
a) Bromopropylene (18.15 g, 0.15 mol) and DMSO (60 mL) were added into a reaction flask, cooled to 0°C, added with glycol (37.26 g, 0.6 mol), DMSO (120 mL), KOH (10.11 g, 0.18 mol) and water (30 mL). The mixture was reacted for 1 hour at the temperature, and then heated to room temperature to react and stir for 18 hours. The reaction solution was added with water (30 mL), and extracted with DCM for 3 times. Organic phases were combined, washed with water for 2 times, dried with anhydrous sodium sulfate, filtered, concentrated under a reduced pressure at about 0°C, and finally purified by column chromatography (eluent: PE: PA = 10:1) to obtain a product **3-1** (6.30 g, yield 25%).
b) *P*-nitrophenyl chloroformate (1.0 g, 4.90 mmol) and a solvent DCM (20.0 mL) were added into a reaction flask, cooled to 0°C, added with the product **3-1** (0.50 g, 4.90 mmol) and pyridine (0.41 g, 5.14 mmol). After the mixture was heated to room temperature to react and stir for 18 hours, the reaction solution was added with saturated sodium carbonate solution (10 mL), and extracted with DCM (50 mL) for 3 times. Organic phases were combined, washed with water for 2 times, dried with anhydrous sodium sulfate, filtered, concentrated under a reduced pressure, and purified by column chromatography (eluent: PE: PA = 2:1) to obtain a product **3-2** (1.00 g, yield 76%).
c) The product **3-2** (1.00 g, 3.74 mmol) and Fmoc-lysine hydrochloride (1.38 g, 3.40 mmol) were added into a reaction flask, added with a solvent dioxane (15 mL) and water (5 mL), and then added with triethylamine (0.86 g, 8.50 mmol). The mixture was reacted at room temperature for 24 hours, added with an with an appropriate amount of 1 M HCl solution to adjust the pH value to be about 2, and extracted with DCM, concentrated under a reduced pressure, and purified by column chromatography (eluent: DCM: MeOH = 30:1) to obtain a product **3-3** (1.01 g, yield 54%).
d) The product **3-3** (6.80 g, 13.69 mmol), ferric sulfate (8.21 g, 20.54 mmol) and palladium chloride (0.48 g, 2.74 mmol) were added into a two-neck reaction flask, added with a solvent acetonitrile (60 mL) and water (10 mL). After the mixture was refluxed at 80°C for 48 hours, the mixture was concentrated under a reduced pressure, extracted with DCM for 3 times, and purified by column chromatography (eluent: DCM: MeOH = 20:1) to obtain a product **3-4** (2.10 g, yield 30%).
e) The product **3-4** (8.00 g, 15.61 mmol) was dissolved in DCM (150 mL) in a reaction flask, added with piperidine (4.00 g, 46.82 mmol), reacted at room temperature for 3 hours, concentrated under a reduced pressure, and purified by column chromatography (eluent: MeOH: H₂O = 40:10:1) to obtain the target product NOPK **3-5** (2.80 g, yield 62%).

¹H-NMR (400 MHz, heavy water) δ 4.41 - 4.25 (m, 2H), 4.23 - 4.10 (m, 2H), 3.85 - 3.66 (m, 2H), 3.66 - 3.60 (m, 1H), 3.06 (t, *J* = 8.4 Hz, 2H), 2.07 (s, 3H), 1.85 - 1.70 (m, 2H), 1.54-1.41 (m, 2H), 1.38 - 1.24 (m, 2H).

### Example 4

Recombinant human growth hormones were prepared in a prokaryotic expression system with NOBK, NOHK and NOPK prepared in Examples 1 to 3, and PEG site-specific conjugates were prepared.

### (1) Acquisition of helper plasmid

A helper plasmid pSupAR-MbPylRS was purchased from the plasmid preservation organization Addgene (catalog number #91705). The plasmid could express a tRNA specifically recognizing an unnatural amino acid derived from pyrrolysine in *Escherichia coli* and a tRNA synthetase, and the helper plasmid was extracted after shake-flask culture in an LB culture medium added with 37.5 mg/L chloramphenicol.

### (2) Construction of expression plasmid of recombinant human growth hormone containing termination codons in reading frame

An mRNA sequence of a coding gene of a Homo sapiens growth hormone (with an amino acid sequence as shown in SEQ ID NO: 1) was obtained from the database of the National Center for Biotechnology Information of the United States, a purification tag consisting of six histidines was added to the C-terminal of the translated protein thereof. Meanwhile, the codons of the amino acid at the 107^{th} site in SEQ ID NO: 1 was changed to amber codons (TAG), and then the complete DNA sequence was synthesized by whole gene synthesis to obtain a gene sequence (SEQ ID NO: 2) of the recombinant human growth hormone.

The amino acid sequence (SEQ ID NO: 1) of the Homo sapiens growth hormone was as follows:

The amino acid sequence (SEQ ID NO: 2) of the recombinant human growth hormone was as follows:

ATGTTTCCTACTATACCACTATCTCGTCTATTCGATAACGCTATGCTTCGGGCCC
ATCGTCTTCATCAGCTGGCCTTTGACACCTACCAGGAGTTTGAAGAAGCCTATATCCC
AAAGGAACAGAAGTATTCATTCCTGCAGAACCCCCAGACCTCCCTCTGTTTCTCAGA
GTCTATTCCGACACCCTCCAACAGGGAGGAAACACAACAGAAATCCAACCTAGAGC
TGCTCCGCATCTCCCTGCTGCTCATCCAGTCGTGGCTGGAGCCCGTGCAGTTCCTCAG
GAGTGTCTTCGCCAACAGCCTGGTGTACGGCGCCTCTTAGAGCAACGTCTATGACCT
CCTAAAGGACCTAGAGGAAGGCATCCAAACGCTGATGGGGAGGCTGGAAGATGGCA
GCCCCCGGACTGGGCAGATCTTCAAGCAGACCTACAGCAAGTTCGACACAAACTCAC
ACAACGATGACGCACTACTCAAGAACTACGGGCTGCTCTACTGCTTCAGGAAGGACA
TGGACAAGGTCGAGACATTCCTGCGCATCGTGCAGTGCCGCTCTGTGGAGGGCAGCT
GTGGCTTCCTCGAGCACCACCACCACCACCACTAA

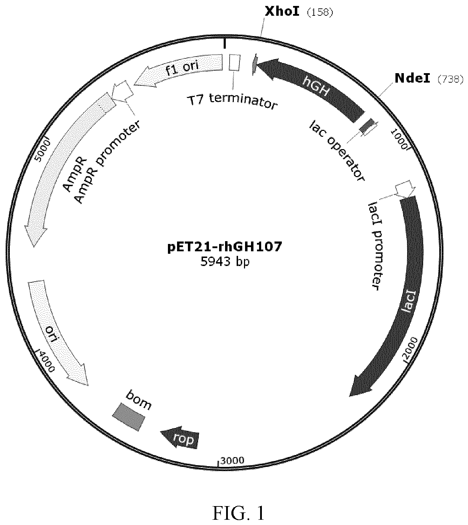

The gene sequence (SEQ ID NO: 2) of the recombinant human growth hormone was cloned between two enzyme cutting sites *Nde* I and *Xho* I of pET21a (Novagen, catalog number #69740-3) through one-step subcloning to obtain an expression plasmid pET21-rhGH107, which was confirmed to be consistent with the expected sequence by sequencing. The pET21-rhGH107 could be used to express the recombinant human growth hormone with the codons of the amino acid at the 107^{th} site changed to the amber codons, and the C-terminal of the protein contained the purification tag consisting of six histidines. The profile of the expression plasmid pET21-rhGH107 of the recombinant human growth hormone was shown in FIG. 1.

### (3) Acquisition of target expression strain

The helper plasmid pSupAR-MbPylRS and the expression plasmid pET21-rhGH107 above were co-transformed into competent cells of *Escherichia coli* OrigamiB (DE3) (Novagen, catalog number #70911-3), and a double-resistant strain was obtained by screening with an LB medium containing 100 mg/L ampicillin and 37.5 mg/L chloramphenicol, i.e., an expression strain of the recombinant human growth hormone.

### (4) Expression of recombinant protein inserted with unnatural amino acid

The screened expression strain of the recombinant human growth hormone was inoculated into four parts of 2×YT medium (16 g/L yeast extract, 10 g/L tryptone and 5 g/LNaCl, containing 100 mg/L ampicillin and 37.5 mg/L chloramphenicol), and cultured at 37°C until OD600 of the bacterial liquid was 2.0 ± 0.2. Four aliquots were respectively added with IPTG (with a final concentration of 1 mM) and arabinose (with a final concentration of 0.2%), wherein the first aliquot was added with NOBK (with a final concentration of 1 mM), the second aliquot was added with NOHK (with a final concentration of 1 mM), the third aliquot was added with NOPK (with a final concentration of 1 mM), and the fourth aliquot was not added with the unnatural amino acid as a negative control. After the four aliquots were cultured at 37°C to induce expression for 5 hours to 6 hours, 1 ml of each culture solution was taken, centrifuged at 10,000 rpm for 1 minute, and resuspended with PBS until OD600 was 10, and each aliquot was taken for SDS-PAGE electrophoresis. The SDS-PAGE electrophoretogram of each strain referred to FIG. 2. Results in FIG. 2 showed that the expression strains respectively added with the three unnatural amino acids could express the target protein.

### (5) Purification of recombinant protein inserted with unnatural amino acid

Each aliquot after the induced expression above was centrifuged at 10,000 rpm for 5 minutes, a precipitate was taken and added with an NTA Buffer (20 mM Tris-HCl at pH 7.9, 0.5 M NaCl and 10% glycerol) with 1/20 cell growth volume and PMSF with a final concentration of 1 mM, and the cells were homogenized and disrupted by ultrasonic wave. The solution was centrifuged at 10,000 rpm for 20 minutes, a supernatant was taken, and a target protein with a His tag was adsorbed by a Ni-NTA chromatography column, and then eluted with an eluent NTA Buffer (20 mM Tris-HCl at pH 7.9, 0.5 M NaCl, 10% glycerol and 250 mM imidazole) to obtain a target protein sample (i.e., the recombinant protein inserted with the unnatural amino acid) with a purity of about 90%. Each sample was placed in a 10 K nitrocellulose dialysis bag, and dialyzed in a PBS buffer at pH 7.0 (200 ml of dialysate per 1 ml of protein solution) overnight, and the dialysate was changed once. The protein solution after dialysis was collected, and the protein concentration was detected by SDS-PAGE.

### (6) Coupling reaction between recombinant protein inserted with unnatural amino acid and PEG

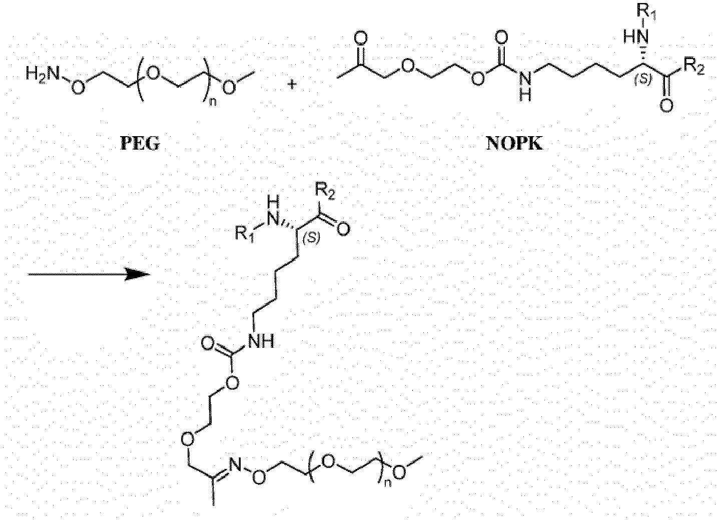

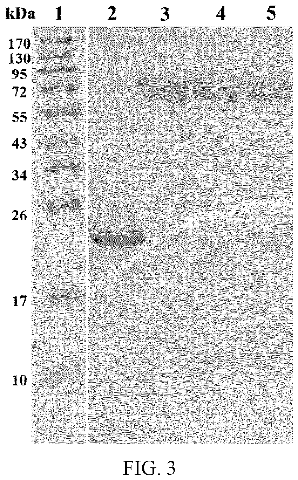

As shown in the synthetic route above (wherein the direction from of R₁ to R₂ was the direction from the N-terminal to the C-terminal of the amino acid sequence):
Taking an oximation reaction of 30 KD aminoxy PEG coupled with the recombinant protein inserted with the unnatural amino acid as an example, the coupling reaction was operated as follows: before the coupling reaction, the target protein obtained above was adjusted to be 0.5 mg/ml with a PBS buffer at pH 7.0, added with a 30 KD aminoxy PEG solid (from Beijing Jenkem Technology Co., Ltd.) according to 1:15 (a molar ratio of the recombinant protein to the aminoxy PEG), and fully shaken for dissolution to obtain a clear and transparent solution. Subsequently, the reaction solution was sealed, and shaken in a constant temperature shaker (25°C, 100 rpm). The coupling situation was analyzed by SDS-PAGE 48 hours later, as shown in FIG. 3. Results in FIG. 3 showed that the three target proteins all achieved 30 KD PEG coupling, and further indicated that the three unnatural amino acids above were inserted into the target proteins. Coupling products of the recombinant protein inserted with the unnatural amino acid coupled with PEG were abbreviated as "PEG-rhGH".

### (7) Purification of PEG-rhGH

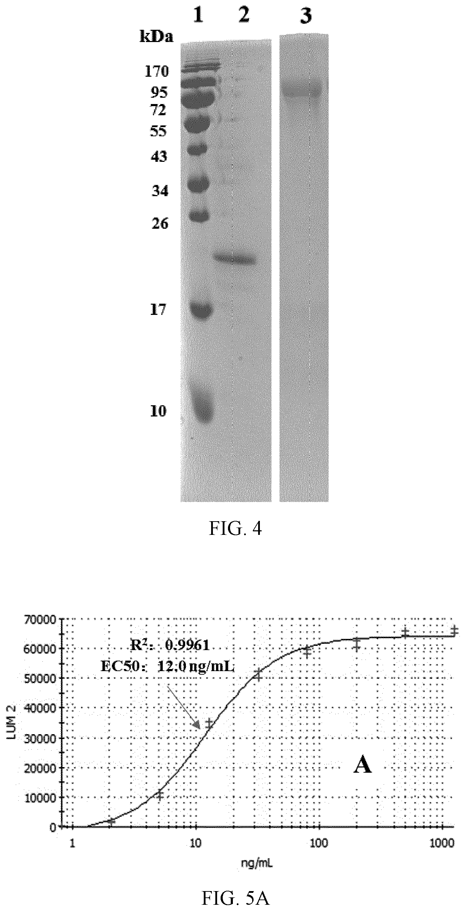

The PEG-rhGH after coupling was separated and purified by a Butyl HP hydrophobic chromatography column (loading buffer: 50 mM NaH₂PO₄ · 2H₂O, and 0.8 M (NH₄)₂SO₄ at pH 8.5; elution buffer: 20 mM Tris-HCl at pH 8.5, and 40 times column volume gradient elution) to obtain pure PEG-rhGH of an electrophoresis pure grade (>95%). Taking PEG-rhGH containing NOPK as example, the electrophoregram was shown in FIG. 4.

Other PEG-rhGH containing the unnatural amino acid of the present invention also reached the electrophoresis pure grade after purification.

### (8) Activity analysis of PEG-rhGH

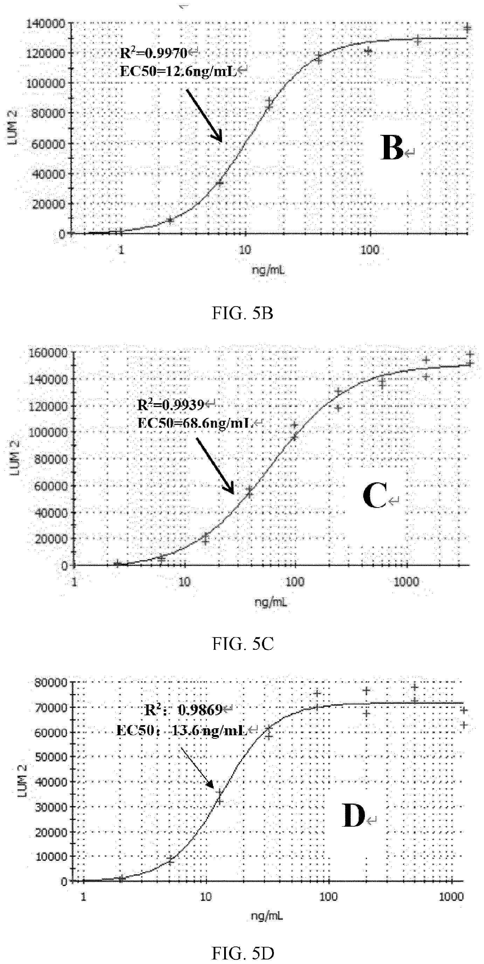

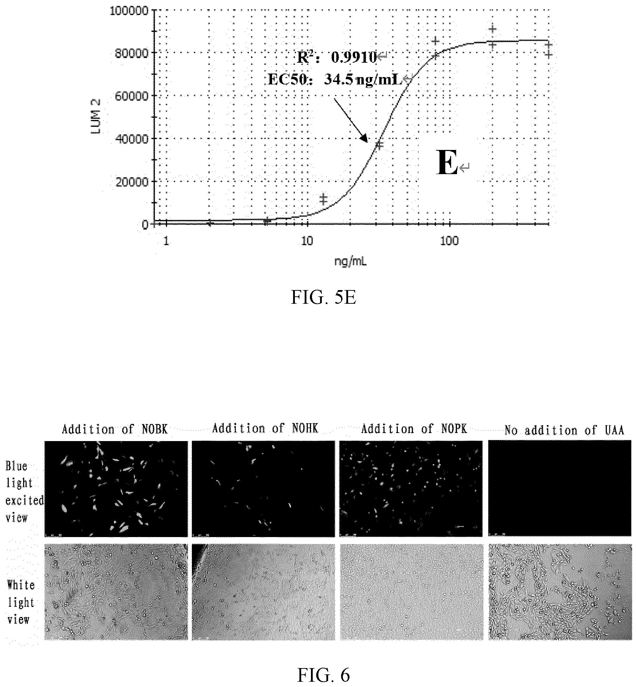

The specific process was as follows: HEK293-GHR cells (cell strains referred to Chinese patent: 2020112649827) were cultured with a DMEM (Gibco, catalog number 11995040) medium containing 0.1 mg/mL hygromycin B (Sangon Biotech (Shanghai) Co., Ltd., catalog number A600230-0001), 0.75 mg/mL G418 (Sangon Biotech (Shanghai) Co., Ltd., catalog number A600958-0005) and 10% fetal bovine serum (Gibco, catalog number 10099141) at 37°C in 5% CO₂ to a sufficient amount, and 9×10⁴ HEK293-GHR cells were inoculated into each well of a black 96-well cell culture plate (Corning, catalog number 3904), with 90 µL/well, and cultured in a starved manner at 37°C in 5% CO₂ for 16 hours. A rhGH standard product (purchased from National Institutes for Food and Drug Control, referred to as "NIFDC"), and samples of rhGH containing NOPK (i.e., rhGH (NOPK)), PEG-rhGH of PEG-modified NOPK (i.e., PEG-rhGH (NOPK)), a commercially available rhGH drug Jintropin^{®} (Changchun GeneScience Pharmaceutical Co., Ltd.) and a commercially available PEG-rhGH drug Polyethylene Glycol Recombinant Human Somatropin Injection^{®} (Changchun GeneScience Pharmaceutical Co., Ltd.) were diluted in gradient, with a total of 9 concentrations, and cultured at 37°C in 5% CO₂ for 4 hours. 50 µL of ONE-Glo^{™} (Promega, catalog number E6120) detection reagent was added into each well, and the chemiluminescence value was read by a microplate reader. The curve was fitted according to the sample concentration and the reading value of the microplate reader, and EC50 was calculated. Results were shown in FIG. 5A to FIG. 5E and Table 1. The results showed that the cell activity of each site before coupling was equivalent to that of the rhGH standard product from NIFDC. The cell activity after coupling was reduced, which was about 35% of that of the standard product, and the change rule of biological activity was consistent with that of PEG-rhGH on the market.

Change rules of biological activity of other PEG-rhGH containing the unnatural amino acid of the present invention after coupling were also consistent with that of PEG-rhGH on the market.

**Table 1**

| Name of sample | EC50 (ng/mL) | Relative activity (%) |
|---|---|---|
| rhGH standard product of NIFDC | 12.0 | 100% |
| rhGH (NOPK) | 13.6 | 88% |
| pEG-rhGH (NOPK) | 34.5 | 35% |
| Jintropin^{®} | 12.6 | 95% |
| Polyethylene Glycol Recombinant Human Somatropin Injection^{®} | 68.6 | 18% |

### Example 5

Recombinant GFP proteins were prepared in a eukaryotic expression system with unnatural amino acids NOBK, NOHK and NOPK prepared in Examples 1 to 3.

### (1) Acquisition of helper plasmid

A helper plasmid pCMV-MbPylRS was purchased from the plasmid preservation organization addgene (catalog number #91706), and the plasmid encoded an aminoacyl tRNA synthetase specifically recognizing an unnatural amino acid derived from pyrrolysine in mammalian cells and a corresponding tRNA (recognizing the amber codons UAG).

### (2) Construction of expression vector of green fluorescent protein containing amber codons in gene reading frame

An expression vector expressing a wild-type green fluorescent protein (with a gene coding sequence shown in SEQ ID NO: 3) containing a purification tag was subjected to point mutation to obtain an expression plasmid pEGFP40 with the codons of the amino acid at the 40^{th} site in the reading frame mutated into amber codons, and the complete sequence of the expression plasmid pEGFP40 was shown in SEQ ID NO: 4.

The gene sequence (SEQ ID NO: 3) of the wild-type green fluorescent protein containing the purification tag was as follows:

ATGGTGAGCAAGGGCGAGGAGCTGTTCACCGGGGTGGTGCCCATCCTGGTCGAG
CTGGACGGCGACGTAAACGGCCACAAGTTCAGCGTGTCCGGCGAGGGCGAGGGCGA
TGCCACCTAGGGCAAGCTGACCCTGAAGTTCATCTGCACCACCGGCAAGCTGCCCGT
GCCCTGGCCCACCCTCGTGACCACCCTGACCTACGGCGTGCAGTGCTTCAGCCGCTAC
CCCGACCACATGAAGCAGCACGACTTCTTCAAGTCCGCCATGCCCGAAGGCTACGTC
CAGGAGCGCACCATCTTCTTCAAGGACGACGGCAACTACAAGACCCGCGCCGAGGTG
AAGTTCGAGGGCGACACCCTGGTGAACCGCATCGAGCTGAAGGGCATCGACTTCAAG
GAGGACGGCAACATCCTGGGGCACAAGCTGGAGTACAACTACAACAGCCACAACGT
CTATATCATGGCCGACAAGCAGAAGAACGGCATCAAGGTGAACTTCAAGATCCGCCA
CAACATCGAGGACGGCAGCGTGCAGCTCGCCGACCACTACCAGCAGAACACCCCCAT
CGGCGACGGCCCCGTGCTGCTGCCCGACAACCACTACCTGAGCACCCAGTCCGCCCT
GAGCAAAGACCCCAACGAGAAGCGCGATCACATGGTCCTGCTGGAGTTCGTGACCGC
CGCCGGGATCACTCTCGGCATGGACGAGCTGTACAAGAAGTACGAAGATTACAAGGA
TGACGACGATAAGTAA

The gene (SEQ ID NO: 4) of the expression plasmid pEGFP40 was as follows:

GTTAGGCGTTTTGCGCTGCTTCGCGATGTACGGGCCAGATATACGCGTTGACATTG
ATTATTGACTAGTTATTAATAGTAATCAATTACGGGGTCATTAGTTCATAGCCCATATATG
GAGTTCCGCGTTACATAACTTACGGTAAATGGCCCGCCTGGCTGACCGCCCAACGACC
CCCGCCCATTGACGTCAATAATGACGTATGTTCCCATAGTAACGCCAATAGGGACTTTC
CATTGACGTCAATGGGTGGAGTATTTACGGTAAACTGCCCACTTGGCAGTACATCAAG
TGTATCATATGCCAAGTACGCCCCCTATTGACGTCAATGACGGTAAATGGCCCGCCTGG
CATTATGCCCAGTACATGACCTTATGGGACTTTCCTACTTGGCAGTACATCTACGTATTA
GTCATCGCTATTACCATGGTGATGCGGTTTTGGCAGTACATCAATGGGCGTGGATAGCG
GTTTGACTCACGGGGATTTCCAAGTCTCCACCCCATTGACGTCAATGGGAGTTTGTTT
TGGCACCAAAATCAACGGGACTTTCCAAAATGTCGTAACAACTCCGCCCCATTGACGC
AAATGGGCGGTAGGCGTGTACGGTGGGAGGTCTATATAAGCAGAGCTCGTTTAGTGAA
CCGTCAGATCGCCTGGAGACGCCATCCACGCTGTTTTGACCTCCATAGAAGACACCGG
GACCGATCCAGCCTCCGGACTCTAGAGGATCCCCGCCACCATGGTGAGCAAGGGCGA
GGAGCTGTTCACCGGGGTGGTGCCCATCCTGGTCGAGCTGGACGGCGACGTAAACGG
CCACAAGTTCAGCGTGTCCGGCGAGGGCGAGGGCGATGCCACCTAGGGCAAGCTGA
CCCTGAAGTTCATCTGCACCACCGGCAAGCTGCCCGTGCCCTGGCCCACCCTCGTGA
CCACCCTGACCTACGGCGTGCAGTGCTTCAGCCGCTACCCCGACCACATGAAGCAGC
ACGACTTCTTCAAGTCCGCCATGCCCGAAGGCTACGTCCAGGAGCGCACCATCTTCTT
CAAGGACGACGGCAACTACAAGACCCGCGCCGAGGTGAAGTTCGAGGGCGACACCC
TGGTGAACCGCATCGAGCTGAAGGGCATCGACTTCAAGGAGGACGGCAACATCCTGG
GGCACAAGCTGGAGTACAACTACAACAGCCACAACGTCTATATCATGGCCGACAAGC

AGAAGAACGGCATCAAGGTGAACTTCAAGATCCGCCACAACATCGAGGACGGCAGC
GTGCAGCTCGCCGACCACTACCAGCAGAACACCCCCATCGGCGACGGCCCCGTGCTG
CTGCCCGACAACCACTACCTGAGCACCCAGTCCGCCCTGAGCAAAGACCCCAACGAG
AAGCGCGATCACATGGTCCTGCTGGAGTTCGTGACCGCCGCCGGGATCACTCTCGGCA
TGGACGAGCTGTACAAGAAGTACGAAGATTACAAGGATGACGACGATAAGTAAGAAT
TCCGGAAGGGTTCGATCCCTACCGGTTAGTAATGAGTTTAAACGGGGGAGGCTAACTG
AAACACGGAAGGAGACAATACCGGAAGGAACCCGCGCTATGACGGCAATAAAAAGA
CAGAATAAAACGCACGGGTGTTGGGTCGTTTGTTCATAAACGCGGGGTTCGGTCCCA
GGGCTGGCACTCTGTCGATACCCCACCGAGACCCCATTGGGGCCAATACGCCCGCGTT
TCTTCCTTTTCCCCACCCCACCCCCCAAGTTCGGGTGAAGGCCCAGGGCTCGCAGCC
AACGTCGGGGCGGCAGGCCCTGCCATAGCAGATCTGCGCAGCTGGGGCTCTAGGGGG
TATCCCCACGCGCCCTGTAGCGGCGCATTAAGCGCGGCGGGTGTGGTGGTTACGCGCA
GCGTGACCGCTACACTTGCCAGCGCCCTAGCGCCCGCTCCTTTCGCTTTCTTCCCTTCC
TTTCTCGCCACGTTCGCCGGCTTTCCCCGTCAAGCTCTAAATCGGGGCATCCCTTTAGG
GTTCCGATTTAGTGCTTTACGGCACCTCGACCCCAAAAAACTTGATTAGGGTGATGGT
TCACGTAGTGGGCCATCGCCCTGATAGACGGTTTTTCGCCCTTTGACGTTGGAGTCCA
CGTTCTTTAATAGTGGACTCTTGTTCCAAACTGGAACAACACTCAACCCTATCTCGGTC
TATTCTTTTGATTTATAAGGGATTTTGGGGATTTCGGCCTATTGGTTAAAAAATGAGCTG
ATTTAACAAAAATTTAACGCGAATTAATTCTGTGGAATGTGTGTCAGTTAGGGTGTGG
AAAGTCCCCAGGCTCCCCAGCAGGCAGAAGTATGCAAAGCATGCATCTCAATTAGTC
AGCAACCAGGTGTGGAAAGTCCCCAGGCTCCCCAGCAGGCAGAAGTATGCAAAGCAT
GCATCTCAATTAGTCAGCAACCATAGTCCCGCCCCTAACTCCGCCCATCCCGCCCCTAA
CTCCGCCCAGTTCCGCCCATTCTCCGCCCCATGGCTGACTAATTTTTTTTATTTATGCAG
AGGCCGAGGCCGCCTCTGCCTCTGAGCTATTCCAGAAGTAGTGAGGAGGCTTTTTTGG
AGGCCTAGGCTTTTGCAAAAAGCTCCCGGGAGCTTGTATATCCATTTTCGGATCTGATC
AAGAGACAGGATGAGGATCGTTTCGCATGATTGAACAAGATGGATTGCACGCAGGTT
CTCCGGCCGCTTGGGTGGAGAGGCTATTCGGCTATGACTGGGCACAACAGACAATCG
GCTGCTCTGATGCCGCCGTGTTCCGGCTGTCAGCGCAGGGGCGCCCGGTTCTTTTTGT
CAAGACCGACCTGTCCGGTGCCCTGAATGAACTGCAGGACGAGGCAGCGCGGCTATC
GTGGCTGGCCACGACGGGCGTTCCTTGCGCAGCTGTGCTCGACGTTGTCACTGAAGC
GGGAAGGGACTGGCTGCTATTGGGCGAAGTGCCGGGGCAGGATCTCCTGTCATCTCA
CCTTGCTCCTGCCGAGAAAGTATCCATCATGGCTGATGCAATGCGGCGGCTGCATACG
CTTGATCCGGCTACCTGCCCATTCGACCACCAAGCGAAACATCGCATCGAGCGAGCAC
GTACTCGGATGGAAGCCGGTCTTGTCGATCAGGATGATCTGGACGAAGAGCATCAGG
GGCTCGCGCCAGCCGAACTGTTCGCCAGGCTCAAGGCGCGCATGCCCGACGGCGAG
GATCTCGTCGTGACCCATGGCGATGCCTGCTTGCCGAATATCATGGTGGAAAATGGCC

GCTTTTCTGGATTCATCGACTGTGGCCGGCTGGGTGTGGCGGACCGCTATCAGGACAT
AGCGTTGGCTACCCGTGATATTGCTGAAGAGCTTGGCGGCGAATGGGCTGACCGCTTC
CTCGTGCTTTACGGTATCGCCGCTCCCGATTCGCAGCGCATCGCCTTCTATCGCCTTCT
TGACGAGTTCTTCTGAGCGGGACTCTGGGGTTCGCGAAATGACCGACCAAGCGACGC
CCAACCTGCCATCACGAGATTTCGATTCCACCGCCGCCTTCTATGAAAGGTTGGGCTT
CGGAATCGTTTTCCGGGACGCCGGCTGGATGATCCTCCAGCGCGGGGATCTCATGCTG
GAGTTCTTCGCCCACCCCAACTTGTTTATTGCAGCTTATAATGGTTACAAATAAAGCAA
TAGCATCACAAATTTCACAAATAAAGCATTTTTTTCACTGCATTCTAGTTGTGGTTTGT
CCAAACTCATCAATGTATCTTATCATGTCTGTATACCGTCGACCTCTAGCTAGAGCTTGG
CGTAATCATGGTCATAGCTGTTTCCTGTGTGAAATTGTTATCCGCTCACAATTCCACAC
AACATACGAGCCGGAAGCATAAAGTGTAAAGCCTGGGGTGCCTAATGAGTGAGCTAA
CTCACATTAATTGCGTTGCGCTCACTGCCCGCTTTCCAGTCGGGAAACCTGTCGTGCC
AGCTGCATTAATGAATCGGCCAACGCGCGGGGAGAGGCGGTTTGCGTATTGGGCGCT
CTTCCGCTTCCTCGCTCACTGACTCGCTGCGCTCGGTCGTTCGGCTGCGGCGAGCGGT
ATCAGCTCACTCAAAGGCGGTAATACGGTTATCCACAGAATCAGGGGATAACGCAGGA
AAGAACATGTGAGCAAAAGGCCAGCAAAAGGCCAGGAACCGTAAAAAGGCCGCGTT
GCTGGCGTTTTTCCATAGGCTCCGCCCCCCTGACGAGCATCACAAAAATCGACGCTCA
AGTCAGAGGTGGCGAAACCCGACAGGACTATAAAGATACCAGGCGTTTCCCCCTGGA
AGCTCCCTCGTGCGCTCTCCTGTTCCGACCCTGCCGCTTACCGGATACCTGTCCGCCTT
TCTCCCTTCGGGAAGCGTGGCGCTTTCTCAATGCTCACGCTGTAGGTATCTCAGTTCG
GTGTAGGTCGTTCGCTCCAAGCTGGGCTGTGTGCACGAACCCCCCGTTCAGCCCGAC
CGCTGCGCCTTATCCGGTAACTATCGTCTTGAGTCCAACCCGGTAAGACACGACTTATC
GCCACTGGCAGCAGCCACTGGTAACAGGATTAGCAGAGCGAGGTATGTAGGCGGTGC
TACAGAGTTCTTGAAGTGGTGGCCTAACTACGGCTACACTAGAAGGACAGTATTTGGT
ATCTGCGCTCTGCTGAAGCCAGTTACCTTCGGAAAAAGAGTTGGTAGCTCTTGATCCG
GCAAACAAACCACCGCTGGTAGCGGTGGTTTTTTTGTTTGCAAGCAGCAGATTACGC
GCAGAAAAAAAGGATCTCAAGAAGATCCTTTGATCTTTTCTACGGGGTCTGACGCTCA
GTGGAACGAAAACTCACGTTAAGGGATTTTGGTCATGAGATTATCAAAAAGGATCTTC
ACCTAGATCCTTTTAAATTAAAAATGAAGTTTTAAATCAATCTAAAGTATATATGAGTAA
ACTTGGTCTGACAGTTACCAATGCTTAATCAGTGAGGCACCTATCTCAGCGATCTGTCT
ATTTCGTTCATCCATAGTTGCCTGACTCCCCGTCGTGTAGATAACTACGATACGGGAGG
GCTTACCATCTGGCCCCAGTGCTGCAATGATACCGCGAGACCCACGCTCACCGGCTCC
AGATTTATCAGCAATAAACCAGCCAGCCGGAAGGGCCGAGCGCAGAAGTGGTCCTGC
AACTTTATCCGCCTCCATCCAGTCTATTAATTGTTGCCGGGAAGCTAGAGTAAGTAGTT
CGCCAGTTAATAGTTTGCGCAACGTTGTTGCCATTGCTACAGGCATCGTGGTGTCACG
CTCGTCGTTTGGTATGGCTTCATTCAGCTCCGGTTCCCAACGATCAAGGCGAGTTACAT

GATCCCCCATGTTGTGCAAAAAAGCGGTTAGCTCCTTCGGTCCTCCGATCGTTGTCAG
AAGTAAGTTGGCCGCAGTGTTATCACTCATGGTTATGGCAGCACTGCATAATTCTCTTA
CTGTCATGCCATCCGTAAGATGCTTTTCTGTGACTGGTGAGTACTCAACCAAGTCATTC
TGAGAATAGTGTATGCGGCGACCGAGTTGCTCTTGCCCGGCGTCAATACGGGATAATA
CCGCGCCACATAGCAGAACTTTAAAAGTGCTCATCATTGGAAAACGTTCTTCGGGGCG
AAAACTCTCAAGGATCTTACCGCTGTTGAGATCCAGTTCGATGTAACCCACTCGTGCA
CCCAACTGATCTTCAGCATCTTTTACTTTCACCAGCGTTTCTGGGTGAGCAAAAACAG
GAAGGCAAAATGCCGCAAAAAAGGGAATAAGGGCGACACGGAAATGTTGAATACTCA
TACTCTTCCTTTTTCAATATTATTGAAGCATTTATCAGGGTTATTGTCTCATGAGCGGAT
ACATATTTGAATGTATTTAGAAAAATAAACAAATAGGGGTTCCGCGCACATTTCCCCGA
AAAGTGCCACCTGACGTCGACGGATCGGGAGATCTCCCGATCCCCTATGGTCGACTCT
CAGTACAATCTGCTCTGATGCCGCATAGTTAAGCCAGTATCTGCTCCCTGCTTGTGTGT
TGGAGGTCGCTGAGTAGTGCGCGAGCAAAATTTAAGCTACAACAAGGCAAGGCTTGA
CCGACAATTGCATGAAGAATCTGCTTAGG

### (3) Expression of recombinant GFP protein inserted with unnatural amino acid

Chinese hamster ovary cells CHO-K1 (catalog number #CCL-61-ATC) were purchased from American Type Culture Collection (ATCC), and cultured in an adherent manner with an RPMI1640 medium containing 10% fetal bovine serum. The helper plasmid pCMV-MbPylRS and the expression plasmid pEGFP40 of the green fluorescent protein in the above steps were extracted, and transiently transfected with a lipo2000 transfection reagent (invitrogen Company, catalog number #12566014) according to the instructions (cells were inoculated into a 24-well plate according to an inoculation amount of 50,000 cells/well, with a total of 4 wells, and were transfected after inoculation and culture for 24 hours, with 500 ng of plasmid per well). During transfection, the first well was added with NOBK (with a final concentration of 1 mM), the second well was added with NOHK (with a final concentration of 1 mM), the third well was added with NOPK (with a final concentration of 1 mM), and the fourth well was not added with the unnatural amino acid as a negative control. The cells were observed under a fluorescence microscope after 48 hours culturing in a CO₂ incubator, and results showed that there was obvious green fluorescence in the first well to the third well (referring to FIG. 6), which proved that the unnatural amino acid could be inserted into the green fluorescent protein to obtain a complete green fluorescent protein without affecting the function of the fluorescent protein.

### Example 6

Anti-HER2 monoclonal antibodies containing unnatural amino acids were prepared in a eukaryotic expression system with NOBK, NOHK and NOPK prepared in Examples 1 to 3, and conjugates with toxin were prepared.

### (1) Acquisition of helper plasmid

A helper plasmid pCMV-MbPylRS was purchased from the plasmid preservation organization addgene (catalog number 91706), and the plasmid encoded an aminoacyl tRNA synthetase specifically recognizing an unnatural amino acid derived from pyrrolysine in mammalian cells and a corresponding tRNA (recognizing the amber codons UAG).

### (2) Construction of expression vector of anti-HER2 antibody (trastuzumab) containing amber codons in gene reading frame

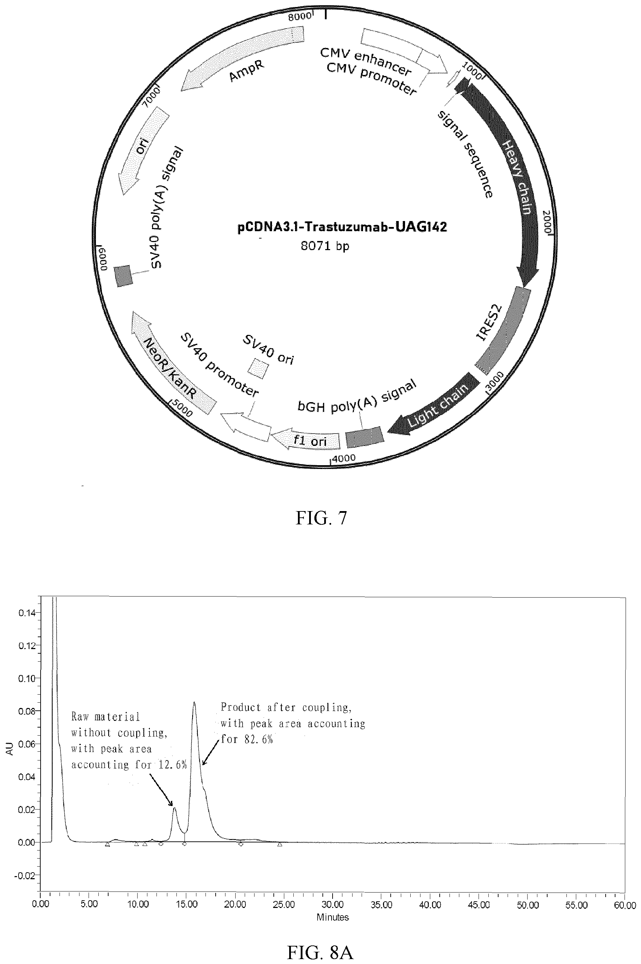

Heavy-chain and light-chain DNAs (corresponding amino acid sequences were SEQ ID NO: 5 and SEQ ID NO: 6 respectively) encoding trastuzumab were synthesized by whole gene synthesis, and subcloned into a eukaryotic expression vector pCDNA3.1+, and then the obtained expression vector was subjected to point mutation to obtain an expression plasmid pCDNA3.1-Trastuzumab-UAG142 with the codons of the amino acid at the 142^{nd} site of the heavy-chain reading frame mutated into amber codons. The profile of the plasmid was shown in FIG. 7, and the complete sequence of the plasmid was shown in SEQ ID NO: 7.

The heavy-chain amino acid sequence (SEQ ID NO: 5) of trastuzumab was as follows:

EVQLVESGGGLVQPGGSLRLSCAASGFNIKDTYIHWVRQAPGKGLEWVARIYPTNG
YTRYADSVKGRFTISADTSKNTAYLQMNSLRAEDTAVYYCSRWGGDGFYAMDYWGQGT
LVTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPGK

The light-chain amino acid sequence (SEQ ID NO: 6) of trastuzumab was as follows:

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC

The gene sequence (SEQ ID NO: 7) of the expression plasmid pCDNA3.1-Trastuzumab-UAG142 was as follows:

GACGGATCGGGAGATCTCCCGATCCCCTATGGTGCACTCTCAGTACAATCTGCTCT
GATGCCGCATAGTTAAGCCAGTATCTGCTCCCTGCTTGTGTGTTGGAGGTCGCTGAGT
AGTGCGCGAGCAAAATTTAAGCTACAACAAGGCAAGGCTTGACCGACAATTGCATGA
AGAATCTGCTTAGGGTTAGGCGTTTTGCGCTGCTTCGCGATGTACGGGCCAGATATACG
CGTTGACATTGATTATTGACTAGTTATTAATAGTAATCAATTACGGGGTCATTAGTTCAT
AGCCCATATATGGAGTTCCGCGTTACATAACTTACGGTAAATGGCCCGCCTGGCTGACC

GCCCAACGACCCCCGCCCATTGACGTCAATAATGACGTATGTTCCCATAGTAACGCCA
ATAGGGACTTTCCATTGACGTCAATGGGTGGAGTATTTACGGTAAACTGCCCACTTGG
CAGTACATCAAGTGTATCATATGCCAAGTACGCCCCCTATTGACGTCAATGACGGTAAA
TGGCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAATGG
GCGTGGATAGCGGTTTGACTCACGGGGATTTCCAAGTCTCCACCCCATTGACGTCAAT
GGGAGTTTGTTTTGGCACCAAAATCAACGGGACTTTCCAAAATGTCGTAACAACTCCG
CCCCATTGACGCAAATGGGCGGTAGGCGTGTACGGTGGGAGGTCTATATAAGCAGAGC
TCTCTGGCTAACTAGAGAACCCACTGCTTACTGGCTTATCGAAATTAATACGACTCACT
ATAGGGAGACCCAAGCTGGCTAGCGTTTAAACTTAAGCTTATGTACCGGATGCAGCTG
CTGAGCTGTATCGCCCTGTCTCTGGCCCTCGTGACCAACAGCGAAGTGCAGCTGGTG
GAAAGCGGCGGAGGACTGGTGCAGCCTGGCGGATCTCTGAGACTGAGCTGTGCCGC
CAGCGGCTTCAACATCAAGGACACCTACATCCACTGGGTGCGCCAGGCCCCTGGCAA
GGGACTGGAATGGGTGGCCAGAATCTACCCCACCAACGGCTACACCAGATACGCCGA
CAGCGTGAAGGGCCGGTTCACCATCAGCGCCGACACCAGCAAGAACACCGCCTACCT
GCAGATGAACAGCCTGCGGGCCGAGGACACCGCCGTGTACTACTGTAGTAGATGGGG
AGGCGACGGCTTCTACGCCATGGACTATTGGGGCCAGGGCACCCTCGTGACAGTGTCT
AGTGCGTAGACCAAGGGGCCCTCGGTCTTCCCCCTGGCACCCTCCTCCAAGAGCACC
TCTGGGGGCACAGCGGCCCTGGGCTGCCTGGTCAAGGACTACTTCCCCGAACCGGTG
ACGGTGTCGTGGAACTCAGGCGCCCTGACCAGCGGCGTGCACACCTTCCCGGCTGTC
CTACAGTCCTCAGGACTCTACTCCCTCAGCAGCGTGGTGACCGTGCCCTCCAGCAGCT
TGGGCACCCAGACCTACATCTGCAACGTGAATCACAAGCCCAGCAACACCAAGGTGG
ACAAGAAAGTTGAGCCCAAATCTTGTGACAAAACTCACACATGCCCACCGTGCCCAG
CACCTGAACTCCTGGGGGGACCGTCAGTCTTCCTCTTCCCCCCAAAACCCAAGGACA
CCCTCATGATCTCCCGGACCCCTGAGGTCACATGCGTGGTGGTGGACGTGAGCCACG
AAGACCCTGAGGTCAAGTTCAACTGGTACGTGGACGGCGTGGAGGTGCATAATGCCA
AGACAAAGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTCTCCAAC
AAAGCCCTCCCAGCCCCCATCGAGAAAACCATCTCCAAAGCCAAAGGGCAGCCCCGA
GAACCACAGGTGTACACCCTGCCCCCATCCCGGGATGAGCTGACCAAGAACCAGGTC
AGCCTGACCTGCCTGGTCAAAGGCTTCTATCCCAGCGACATCGCCGTGGAGTGGGAG
AGCAATGGGCAGCCGGAGAACAACTACAAGACCACGCCTCCCGTGCTGGACTCCGAC
GGCTCCTTCTTCCTCTACAGCAAGCTCACCGTGGACAAGAGCAGGTGGCAGCAGGGG
AACGTCTTCTCATGCTCCGTGATGCATGAGGCTCTGCACAACCACTACACGCAGAAGA
GCCTCTCCCTGTCTCCCGGCAAGTGATAAGGCCGGCCCCTCTCCCTCCCCCCCCCCTA
ACGTTACTGGCCGAAGCCGCTTGGAATAAGGCCGGTGTGCGTTTGTCTATATGTTATTT

TCCACCATATTGCCGTCTTTTGGCAATGTGAGGGCCCGGAAACCTGGCCCTGTCTTCTT
GACGAGCATTCCTAGGGGTCTTTCCCCTCTCGCCAAAGGAATGCAAGGTCTGTTGAAT
GTCGTGAAGGAAGCAGTTCCTCTGGAAGCTTCTTGAAGACAAACAACGTCTGTAGCG
ACCCTTTGCAGGCAGCGGAACCCCCCACCTGGCGACAGGTGCCTCTGCGGCCAAAAG
CCACGTGTATAAGATACACCTGCAAAGGCGGCACAACCCCAGTGCCACGTTGTGAGT
TGGATAGTTGTGGAAAGAGTCAAATGGCTCTCCTCAAGCGTATTCAACAAGGGGCTG
AAGGATGCCCAGAAGGTACCCCATTGTATGGGATCTGATCTGGGGCCTCGGTACACAT
GCTTTACATGTGTTTAGTCGAGGTTAAAAAAACGTCTAGGCCCCCCGAACCACGGGG
ACGTGGTTTTCCTTTGAAAAACACGATGATAATATGGCCACAACCATGTACCGCATGC
AACTCCTGTCTTGCATTGCACTAAGTCTTGCACTTGTCACAAACAGTGATATCCAGATG
ACCCAGAGCCCCAGCAGCCTGTCTGCCAGCGTGGGCGACAGAGTGACCATCACCTGT
AGAGCCAGCCAGGACGTGAACACCGCCGTGGCCTGGTATCAGCAGAAGCCTGGCAA
GGCCCCCAAGCTGCTGATCTACAGCGCCAGCTTCCTGTACAGCGGCGTGCCCAGCAG
ATTCAGCGGCAGCAGATCCGGCACCGACTTCACCCTGACCATCAGCTCCCTGCAGCCC
GAGGACTTCGCCACCTACTACTGCCAGCAGCACTACACCACCCCCCCCACATTTGGCC
AGGGCACCAAGGTGGAAATCAAGCGTACGGTGGCTGCACCATCTGTCTTCATCTTCCC
GCCATCTGATGAGCAGTTGAAATCTGGAACTGCCTCTGTTGTGTGCCTGCTGAATAAC
TTCTACCCCAGAGAAGCCAAAGTGCAGTGGAAGGTGGACAACGCCCTGCAGAGCGG
AAACAGCCAGGAAAGCGTGACAGAGCAGGATTCCAAGGATTCCACATACAGCCTGAG
CAGCACACTGACACTGTCCAAGGCCGACTACGAGAAGCACAAGGTGTACGCCTGCG
AAGTGACACACCAGGGACTGTCCTCCCCTGTGACAAAGAGCTTCAACAGAGGAGAA
TGCTGATGATCTAGAGGGCCCGTTTAAACCCGCTGATCAGCCTCGACTGTGCCTTCTA
GTTGCCAGCCATCTGTTGTTTGCCCCTCCCCCGTGCCTTCCTTGACCCTGGAAGGTGC
CACTCCCACTGTCCTTTCCTAATAAAATGAGGAAATTGCATCGCATTGTCTGAGTAGGT
GTCATTCTATTCTGGGGGGTGGGGTGGGGCAGGACAGCAAGGGGGAGGATTGGGAAG
ACAATAGCAGGCATGCTGGGGATGCGGTGGGCTCTATGGCTTCTGAGGCGGAAAGAA
CCAGCTGGGGCTCTAGGGGGTATCCCCACGCGCCCTGTAGCGGCGCATTAAGCGCGG
CGGGTGTGGTGGTTACGCGCAGCGTGACCGCTACACTTGCCAGCGCCCTAGCGCCCG
CTCCTTTCGCTTTCTTCCCTTCCTTTCTCGCCACGTTCGCCGGCTTTCCCCGTCAAGCT
CTAAATCGGGGGCTCCCTTTAGGGTTCCGATTTAGTGCTTTACGGCACCTCGACCCCA
AAAAACTTGATTAGGGTGATGGTTCACGTAGTGGGCCATCGCCCTGATAGACGGTTTT
TCGCCCTTTGACGTTGGAGTCCACGTTCTTTAATAGTGGACTCTTGTTCCAAACTGGA
ACAACACTCAACCCTATCTCGGTCTATTCTTTTGATTTATAAGGGATTTTGCCGATTTCG
GCCTATTGGTTAAAAAATGAGCTGATTTAACAAAAATTTAACGCGAATTAATTCTGTGG
AATGTGTGTCAGTTAGGGTGTGGAAAGTCCCCAGGCTCCCCAGCAGGCAGAAGTATG
CAAAGCATGCATCTCAATTAGTCAGCAACCAGGTGTGGAAAGTCCCCAGGCTCCCCA

GCAGGCAGAAGTATGCAAAGCATGCATCTCAATTAGTCAGCAACCATAGTCCCGCCCC
TAACTCCGCCCATCCCGCCCCTAACTCCGCCCAGTTCCGCCCATTCTCCGCCCCATGGC
TGACTAATTTTTTTTATTTATGCAGAGGCCGAGGCCGCCTCTGCCTCTGAGCTATTCCA
GAAGTAGTGAGGAGGCTTTTTTGGAGGCCTAGGCTTTTGCAAAAAGCTCCCGGGAGC
TTGTATATCCATTTTCGGATCTGATCAAGAGACAGGATGAGGATCGTTTCGCATGATTG
AACAAGATGGATTGCACGCAGGTTCTCCGGCCGCTTGGGTGGAGAGGCTATTCGGCTA
TGACTGGGCACAACAGACAATCGGCTGCTCTGATGCCGCCGTGTTCCGGCTGTCAGC
GCAGGGGCGCCCGGTTCTTTTTGTCAAGACCGACCTGTCCGGTGCCCTGAATGAACT
GCAGGACGAGGCAGCGCGGCTATCGTGGCTGGCCACGACGGGCGTTCCTTGCGCAGC
TGTGCTCGACGTTGTCACTGAAGCGGGAAGGGACTGGCTGCTATTGGGCGAAGTGCC
GGGGCAGGATCTCCTGTCATCTCACCTTGCTCCTGCCGAGAAAGTATCCATCATGGCT
GATGCAATGCGGCGGCTGCATACGCTTGATCCGGCTACCTGCCCATTCGACCACCAAG
CGAAACATCGCATCGAGCGAGCACGTACTCGGATGGAAGCCGGTCTTGTCGATCAGG
ATGATCTGGACGAAGAGCATCAGGGGCTCGCGCCAGCCGAACTGTTCGCCAGGCTCA
AGGCGCGCATGCCCGACGGCGAGGATCTCGTCGTGACCCATGGCGATGCCTGCTTGC
CGAATATCATGGTGGAAAATGGCCGCTTTTCTGGATTCATCGACTGTGGCCGGCTGGG
TGTGGCGGACCGCTATCAGGACATAGCGTTGGCTACCCGTGATATTGCTGAAGAGCTT
GGCGGCGAATGGGCTGACCGCTTCCTCGTGCTTTACGGTATCGCCGCTCCCGATTCGC
AGCGCATCGCCTTCTATCGCCTTCTTGACGAGTTCTTCTGAGCGGGACTCTGGGGTTC
GAAATGACCGACCAAGCGACGCCCAACCTGCCATCACGAGATTTCGATTCCACCGCC
GCCTTCTATGAAAGGTTGGGCTTCGGAATCGTTTTCCGGGACGCCGGCTGGATGATCC
TCCAGCGCGGGGATCTCATGCTGGAGTTCTTCGCCCACCCCAACTTGTTTATTGCAGC
TTATAATGGTTACAAATAAAGCAATAGCATCACAAATTTCACAAATAAAGCATTTTTTT
CACTGCATTCTAGTTGTGGTTTGTCCAAACTCATCAATGTATCTTATCATGTCTGTATAC
CGTCGACCTCTAGCTAGAGCTTGGCGTAATCATGGTCATAGCTGTTTCCTGTGTGAAAT
TGTTATCCGCTCACAATTCCACACAACATACGAGCCGGAAGCATAAAGTGTAAAGCCT
GGGGTGCCTAATGAGTGAGCTAACTCACATTAATTGCGTTGCGCTCACTGCCCGCTTT
CCAGTCGGGAAACCTGTCGTGCCAGCTGCATTAATGAATCGGCCAACGCGCGGGGAG
AGGCGGTTTGCGTATTGGGCGCTCTTCCGCTTCCTCGCTCACTGACTCGCTGCGCTCG
GTCGTTCGGCTGCGGCGAGCGGTATCAGCTCACTCAAAGGCGGTAATACGGTTATCCA
CAGAATCAGGGGATAACGCAGGAAAGAACATGTGAGCAAAAGGCCAGCAAAAGGCC
AGGAACCGTAAAAAGGCCGCGTTGCTGGCGTTTTTCCATAGGCTCCGCCCCCCTGAC
GAGCATCACAAAAATCGACGCTCAAGTCAGAGGTGGCGAAACCCGACAGGACTATAA
AGATACCAGGCGTTTCCCCCTGGAAGCTCCCTCGTGCGCTCTCCTGTTCCGACCCTGC
CGCTTACCGGATACCTGTCCGCCTTTCTCCCTTCGGGAAGCGTGGCGCTTTCTCATAGC
TCACGCTGTAGGTATCTCAGTTCGGTGTAGGTCGTTCGCTCCAAGCTGGGCTGTGTGC

ACGAACCCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAGGATTAG
CAGAGCGAGGTATGTAGGCGGTGCTACAGAGTTCTTGAAGTGGTGGCCTAACTACGG
CTACACTAGAAGAACAGTATTTGGTATCTGCGCTCTGCTGAAGCCAGTTACCTTCGGA
AAAAGAGTTGGTAGCTCTTGATCCGGCAAACAAACCACCGCTGGTAGCGGTTTTTTTG
TTTGCAAGCAGCAGATTACGCGCAGAAAAAAAGGATCTCAAGAAGATCCTTTGATCTT
TTCTACGGGGTCTGACGCTCAGTGGAACGAAAACTCACGTTAAGGGATTTTGGTCATG
AGATTATCAAAAAGGATCTTCACCTAGATCCTTTTAAATTAAAAATGAAGTTTTAAATC
AATCTAAAGTATATATGAGTAAACTTGGTCTGACAGTTACCAATGCTTAATCAGTGAGG
CACCTATCTCAGCGATCTGTCTATTTCGTTCATCCATAGTTGCCTGACTCCCCGTCGTGT
AGATAACTACGATACGGGAGGGCTTACCATCTGGCCCCAGTGCTGCAATGATACCGCG
AGACCCACGCTCACCGGCTCCAGATTTATCAGCAATAAACCAGCCAGCCGGAAGGGC
CGAGCGCAGAAGTGGTCCTGCAACTTTATCCGCCTCCATCCAGTCTATTAATTGTTGCC
GGGAAGCTAGAGTAAGTAGTTCGCCAGTTAATAGTTTGCGCAACGTTGTTGCCATTGC
TACAGGCATCGTGGTGTCACGCTCGTCGTTTGGTATGGCTTCATTCAGCTCCGGTTCCC
AACGATCAAGGCGAGTTACATGATCCCCCATGTTGTGCAAAAAAGCGGTTAGCTCCTT
CGGTCCTCCGATCGTTGTCAGAAGTAAGTTGGCCGCAGTGTTATCACTCATGGTTATGG
CAGCACTGCATAATTCTCTTACTGTCATGCCATCCGTAAGATGCTTTTCTGTGACTGGT
GAGTACTCAACCAAGTCATTCTGAGAATAGTGTATGCGGCGACCGAGTTGCTCTTGCC
CGGCGTCAATACGGGATAATACCGCGCCACATAGCAGAACTTTAAAAGTGCTCATCAT
TGGAAAACGTTCTTCGGGGCGAAAACTCTCAAGGATCTTACCGCTGTTGAGATCCAGT
TCGATGTAACCCACTCGTGCACCCAACTGATCTTCAGCATCTTTTACTTTCACCAGCGT
TTCTGGGTGAGCAAAAACAGGAAGGCAAAATGCCGCAAAAAAGGGAATAAGGGCGA
CACGGAAATGTTGAATACTCATACTCTTCCTTTTTCAATATTATTGAAGCATTTATCAGG
GTTATTGTCTCATGAGCGGATACATATTTGAATGTATTTAGAAAAATAAACAAATAGGG
GTTCCGCGCACATTTCCCCGAAAAGTGCCACCTGACGTC

### (3) Insertion of unnatural amino acid

HEK293 cells adopted to suspension were inoculated into a Wayne293^{™} medium (Quacell Biotechnology, catalog number A21501) at a density of 0.3×10⁵/mL, and cultured by shaking in a 1 L shake flask loaded with 240 mL of medium under conditions of 120 rpm, 5% CO₂ and 80% humidity. The cells were transfected when the cell density reached about 1×10⁶/mL, the helper plasmid pCMV-MbPylRS and the expression plasmid pCDNA3.1-Trastuzumab-UAG142 described in the steps (1) and (2) were extracted, and the endotoxin was removed for later use. During transfection of each shake flask, 120 µg of expression plasmid and 120 µg of helper plasmid were used and added into a centrifuge tube and diluted to 7.2 mL with 1×PBS buffer, and then 720 µg of PEI transfection reagent (Polyethyleneimine) was added into another centrifuge tube, and added with 1×PBS buffer to dilute to 7.2 mL. The two centrifuge tubes were respectively left to stand for 5 minutes after mixing evenly, and then liquids in the two centrifuge tubes were gently and evenly mixed and allowed to stand for 10 minutes, slowly and dropwise added into 240 mL of cell suspension in the 1 L shake flask by a total of 14.4 mL, continuously and gently shaken during dropwise adding, cultured by shaking under conditions of 120 rpm, 5% CO₂ and 80% humidity for 4 hours, then added with the unnatural amino acid with the final concentration of 1 mM, and continuously cultured under conditions of 120 rpm, 5% CO₂ and 80% humidity for 5 days, and a cell culture supernatant was collected for antibody purification.

### (4) Purification

The cell culture supernatant was purified with 1 ml of HiTrap Protein A prepacked column. The supernatant was eluted with an elution buffer of 100 mmol/L glycine and 200 mmol/L acetate at pH 3.5 to obtain the purified trastuzumab inserted with the unnatural amino acid.

### (5) Coupling

The synthetic route was as follows (taking NOPK as an example):

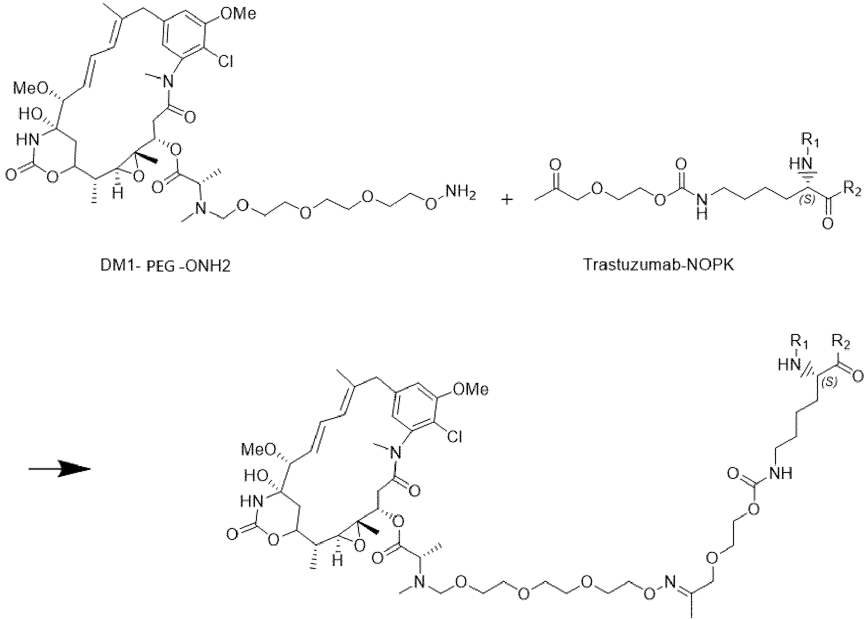

A toxin containing an amino-oxygen terminal group (DM1-PEG-ONH₂) and the purified trastuzumab inserted with the unnatural amino acid (wherein the direction from R₁ to R₂ was the direction from the N-terminal to the C-terminal of the amino acid sequence) were coupled in a site-specific manner through an oximation reaction to obtain a monoclonal antibody-toxin conjugate.

The preparation process of DM1-PEG-ONH₂ was as follows:

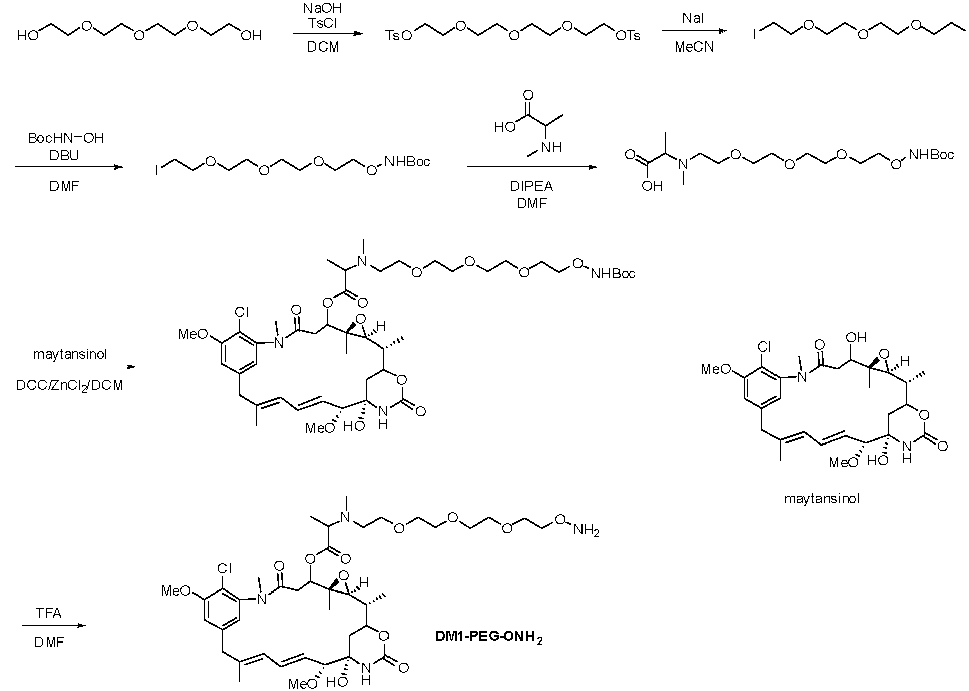

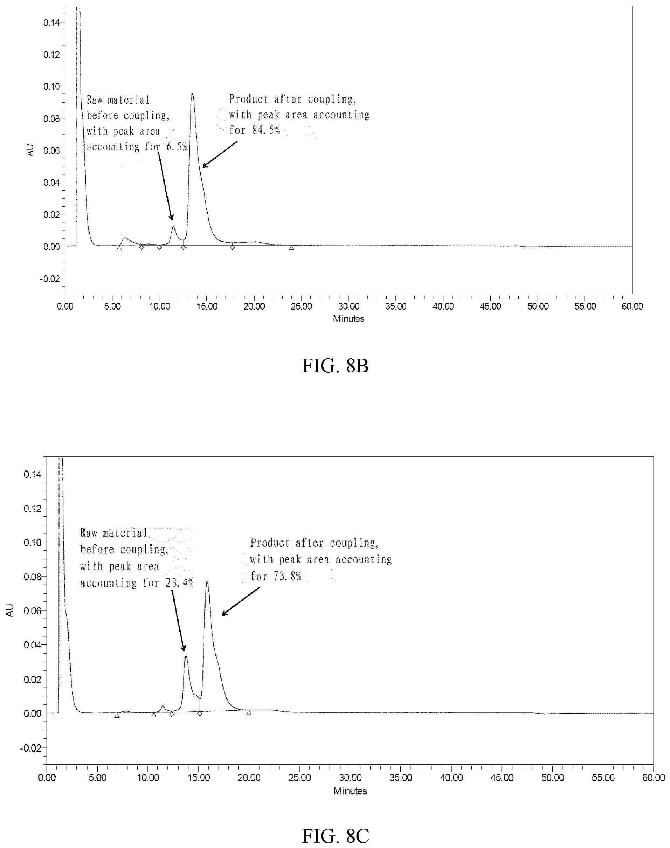

Specific operations of the coupling process were as follows: the purified trastuzumab inserted with the unnatural amino acid was mixed and dissolved with the DM1-PEG-ONH₂ in a molar ratio of 1: 15, then the pH value was adjusted to be 4.0 with 10 M acetic acid, the mixture was shaken on a shaker (25°C, 200 rpm), and sampled 48 hours later, and the reaction situation of trastuzumab was detected by HPLC (HIC-HPLC) based on the hydrophobic chromatography principle. Results showed that 87.4% of trastuzumab containing NOPK was modified by the toxin; 93.5% of trastuzumab containing NOHK was modified by the toxin; and 76.6% of trastuzumab containing NOBK was modified by the toxin, as shown in FIG. 8A to FIG. 8C. Coupling ratio of trastuzumab = 100% - ratio of remaining uncoupled raw materials.

The coupled monoclonal antibody-toxin conjugate was subjected to buffer change through a 50 kDa ultrafiltration centrifuge tube to remove unreacted toxin raw materials, and the changed buffer was 20 mM histidine buffer (pH 6.5).

HIC-HPLC analysis conditions were as follows:
mobile phase A (2 M ammonium sulfate, 75 mM K₂HPO₄, pH 7.2±0.2); and
mobile phase B (75 mM K₂HPO₄, 25% isopropanol, pH 7.2±0.2).

| Chromatographic column | TSK gel Butyl-NPR column, 4.6 × 100mm, 2.5 µm | | |
|---|---|---|---|
| Detection wavelength | 280 nm | | |
| Temperature of column thermerstat | 40°C | | |
| Temperature of sample injector | 5°C | | |
| Flow velocity | 0.5 ml/min | | |
| Sample volume | 10 µl | | |

| | Time (min) | A (%) | B (%) |
|---|---|---|---|
| Gradient elution | 0 | 80 | 20 |
| | 30 | 40 | 60 |
| | 35 | 25 | 75 |
| | 40 | 25 | 75 |
| | 45 | 80 | 20 |
| | 60 | 80 | 20 |

### (6) Activity analysis

BT-474 cell strains (ATCC, catalog number HTB-20) were used to detect the inhibitory effect of the sample on cell proliferation.

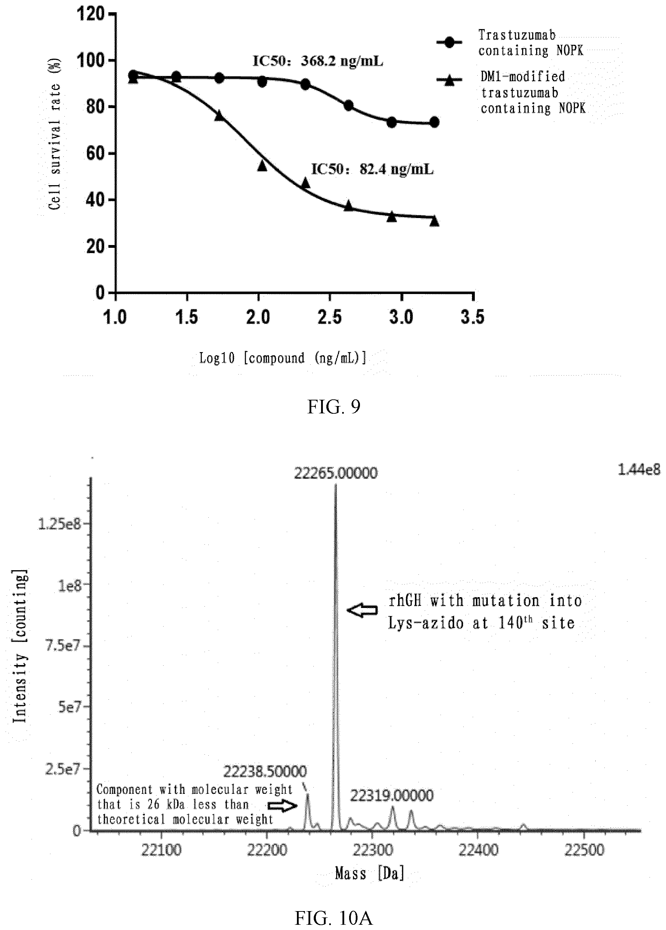

The specific process was as follows: BT-474 cells were cultured with an ATCC Hybri-Care Medium (ATCC, catalog number 46-X) medium containing 10% fetal bovine serum (Gibco, catalog number 10099141) at the condition of 37°C and 5% CO₂ to a sufficient amount, 1.5×10⁴ BT-474 cells were inoculated into each well of a 96-well cell culture plate (Corning, catalog number 3599) (including a vehicle control well), and cell-free medium was added into a blank control well, with 80 µL/well. The trastuzumab containing NOPK and the DM1-modified trastuzumab containing NOPK were gradiently diluted from 17 µg/mL to 0.13 µg/mL, with a total of 8 concentrations, and 2 repeated wells for each concentration. The diluted sample was transferred to the culture plate inoculated with the BT-474 cells with 10 µL/well, and 10 µL of medium was added into the vehicle control well and the blank control well respectively, and cultured at the condition of 37°C and 5% CO₂. On the fifth day, 10 µL of CCK-8 (Beyotime, catalog number C0043) detection reagent was added into each well, and developed at the condition of 37°C and 5% CO₂ for 6 hours, and the optical density was read by a microplate reader at a wavelength of 450 nm. The growth inhibition of the detected sample was calculated by the following formula: growth inhibition (%) = (OD compound - OD blank control) / (OD vehicle control - OD blank control) ×100%. Growth inhibitions of compounds with different concentrations were calculated in Excel, and then the growth inhibition curve was made by GraphPad Prism7 software and IC50 was calculated. Results were shown in FIG. 9. The results showed that the IC50 of the DM1-modified trastuzumab containing NOPK was about 4.5 times lower than that of the trastuzumab containing NOPK, so that the tumor killing effect was significantly improved.

### (7) Stability examination of DM1-modified trastuzumab containing unnatural amino acid

The coupled monoclonal antibody-toxin conjugate was subjected to buffer change through a 50 kDa ultrafiltration centrifuge tube (Millipore, catalog number UFC905024#) to remove unreacted toxin raw materials. The exchanged buffer was 20 mM histidine buffer (pH 6.0). The protein solution was divided into three parts, adjusted to pH 4.0, pH 6.0 and pH 8.0 with 1 M citric acid solution or 1 M Tris solution respectively, placed in a water bath at 25°C, sampled 24 hours, 48 hours and 72 hours later respectively, and analyzed by HIC-HPLC (the detection conditions are the same as above). Results were shown in Table 2.

**Table 2 Stability of DM1-modified trastuzumab containing NOPK at 25°C under different pH values**

| Sampling time | Content of uncoupled trastuzumab containing NOPK/% | | |
|---|---|---|---|
| | pH 4.0 | pH6.0 | pH8.0 |
| 0 h | 12.6 | | |
| 24 h | 57.8 | 19.5 | 13.5 |
| 48 h | 77.3 | 27.6 | 14.7 |
| 72 h | 83.7 | 36.0 | 16.9 |

Results showed that the oxime bond formed in the DM1-modified trastuzumab containing the unnatural amino acid provided by the present invention had the characteristics of reversible decomposition at a low pH, and was relatively stable under neutral and alkaline pH conditions.

### Example 7 Reduction of active group of Lys-azido

### (1) Analysis of molecular weight of product by high-resolution mass spectrometry

According to the expression method of the secretory growth hormone in a reference (Lihua Song, et al., Journal of Biology, 16 (1): 6-8, 1999), an OmpA signal peptide coding sequence was added to the N-terminal of rhGH on the expression vector according to the signal peptide sequence provided in the reference, and in combination with the helper plasmid pSupAR-MbPylRS in Example 4, an OrigamiB (DE3) expression strain of rhGH with K140 codons mutated into amber codons (TAG) was constructed. Natural rhGH with the mutation into Lys-azido at the 140^{th} site was expressed by adding Lys-azido during fermentation, which was named rhGH-Lys-azido-140. Natural rhGH with a mutation into NOPK at the 140^{th} site was expressed by adding NOPK during fermentation, which was named rhGH-NOPK-140. Moreover, purification was carried out by corresponding purification means in the reference above. Complete molecular weights of the rhGH-Lys-azido-140 and the rhGH-NOPK-140 were analyzed through liquid chromatography-mass spectrometry (high-resolution mass spectrometer: XevoG2-XS Q-Tof, Waters Corporation; Ultra-high performance liquid chromatography: UPLC (Acquity UPLC I-Class), Waters Corporation).

As shown in FIG. 10A, a component with a molecular weight that was 26 Da less than the theoretical molecular weight (22,265 Da) appeared in the rhGH-Lys-azido-140 sample, and the component was inferred to be a product of reduction of the azide structure (-N₃) at the terminal of Lys-azido into (-NH₂), which indicated that the azide group was unstable when the commonly used unnatural amino acid Lys-azido was inserted into a protein, and easily reduced to form a reduction product, thus losing coupling activity. As shown in FIG. 10B, the rhGH-NOPK-140 sample did not have a high proportion of impurities, which indicated that the recombinant protein product prepared by NOPK was more stable than that prepared by Lys-azido.

### (2) Comparison of coupling efficiency

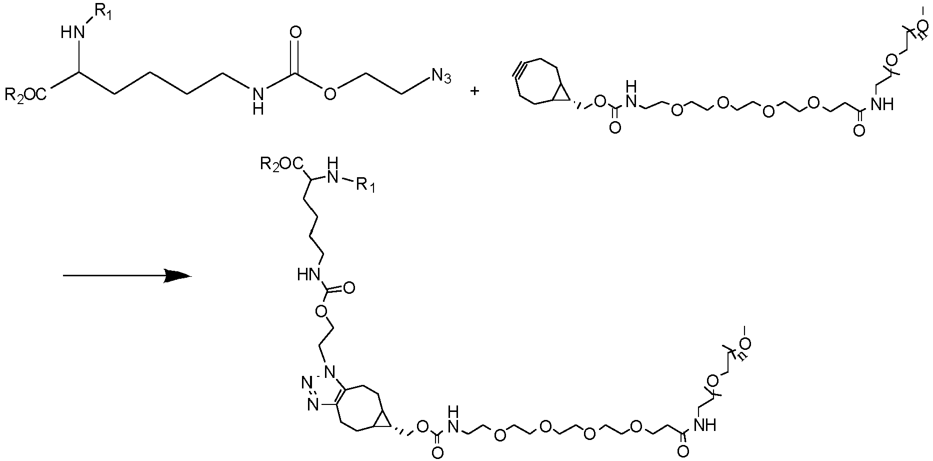

As shown in the above reaction route, 30 KD BCN-PEG (home-made according to the Chinese patent CN112279906A) was coupled with rhGH-Lys-azido-140 in a site-specific manner by Click reaction (wherein the direction from R₁ to R₂ was the direction from the N-terminal to the C-terminal of the amino acid sequence).

The target protein obtained above was adjusted to be 0.5 mg/ml with a PBS buffer at pH 7.0, BCN-PEG powder was added into the rhGH-Lys-azido solution according to 1:15 and 1:25 (molar ratios of recombinant protein to 30 KD BCN-PEG) respectively, and fully shaken for dissolution to obtain a clear and transparent solution, and then the reaction solution was sealed, and shaken to react in a constant temperature shaker (25°C, 70 rpm). Sampling was carried out and reaction results were detected by SDS-PAGE at regular intervals, and the reaction was stopped 72 hours later, with a conversion rate of about 50% to 70%. Reaction results were shown in FIG. 11A.

With reference to Example 4, the 30 KD aminoxy PEG was coupled with the rhGH-NOPK-140 in a site-specific manner by the oximation reaction. Sampling was carried out and reaction results were detected by SDS-PAGE at regular intervals, and the reaction was stopped 48 hours later, with a conversion rate of about 90%. Reaction results were shown in FIG. 11B.

Combined with molecular weight detection results of high-resolution mass spectrometry, it could be judged that the reduction of Lys-azido leaded to the incapability of coupling the product with BCN-PEG, thus reducing the coupling rate. However, when the recombinant protein containing the unnatural amino acid of the present invention was coupled with PEG, the conversion rate was obviously higher than that of the recombinant protein containing the Lys-azido, and the reaction time was also obviously shortened, thus obviously improving reaction efficiency.

Unless otherwise specified, the terms used in the present invention have the meanings commonly understood by those skilled in the art.

The described embodiments of the present invention are for exemplary purposes only, and are not intended to limit the scope of protection of the present invention. Those skilled in the art can make various other substitutions, changes and improvements within the scope of the present invention. Therefore, the present invention is not limited to the above embodiments, but only limited by the claims.

## Claims

1. An unnatural amino acid, wherein the unnatural amino acid is a compound with a structure as shown in formula (I) or an enantiomer thereof, 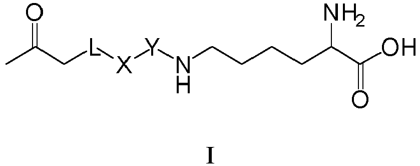
wherein X represents -O-, -S-, -NH- or -CH₂-, Y represents -O-, -S-, -C(O)-, -S(O)- or -CH₂-, and L represents substituted or unsubstituted C0-C20 linear or branched alkylene, wherein one or more -CH₂- are optionally substituted by one or more of -O-, -S-, -NH-, -C(O)- and -S(O)-;
when X represents -O- and Y represents -C(O)-, L does not represent -CH₂CH₂-; and
when L represents a substituent, the substituent is selected from one or more of hydroxyl, sulfhydryl, halogen, nitro, cyano, alkyl, alkenyl, alkynyl, alkoxy, acyl, acylamino, carboxyl, ester, amino, sulfonyl, sulfinyl, cycloalkyl, heterocyclyl, aryl and heteroaryl.

2. The unnatural amino acid according to claim 1, wherein the substituent is selected from one or more of hydroxyl, sulfhydryl, halogen, nitro, cyano, C1-C6 alkyl, C1-C6 alkoxy, acyl, acylamino, carboxyl, ester, amino, sulfonyl, sulfinyl, C3-C8 cycloalkyl, C3-C8 heterocyclyl, C6-C20 aryl and C4-C10 heteroaryl;
preferably, L represents C0-C10 linear or branched alkylene, and one or more -CH₂- are optionally substituted by -O-; and more preferably, L represents C0-C6 linear alkylene; and/or
X represents -O-, -S-, -NH- or -CH₂-; and/or
Y represents-C(O)-.

3. The unnatural amino acid according to claim 1 or 2, wherein the unnatural amino acid is a compound with a structure as shown in formula (I-1), 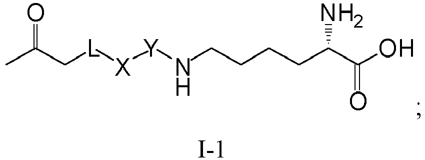 wherein X, Y and L are each independently as defined in claim 1 or 2.

4. The unnatural amino acid according to claim 3, wherein the unnatural amino acid is a compound with a structure as shown in formula (I-2), formula (I-3), formula (I-4) or formula (I-5), 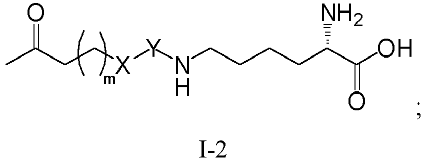 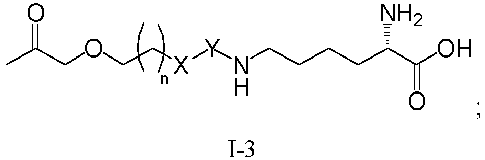 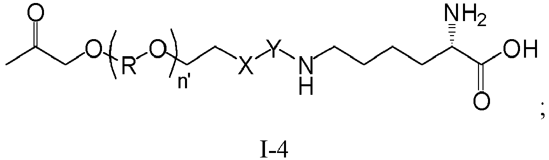 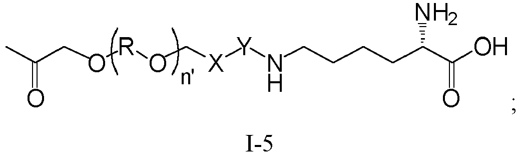
wherein X and Y are each independently as defined in any one of claims 1-3, m and n each independently represents an integer of 0-8, preferably represents an integer of 0-5, and more preferably represents 0, 1, 2 or 3;
when X represents -O- and Y represents -C(O)-, m does not represent 1; and
R represents C1-C4 linear or branched alkylene, and preferably represents -CH₂-, -CH₂CH₂- or -CH₂CH₂CH_{2-;} and n' represents an integer of 0-5, preferably represents an integer of 0-3, and more preferably represents 0, 1 or 2.

5. The unnatural amino acid according to any one of claims 1-4, wherein the unnatural amino acid is a compound with one of the structures as follows,
| | |
|---|---|
| (1) | 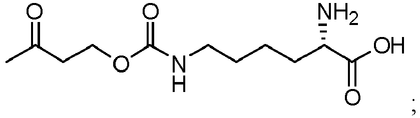 |
| (2) | 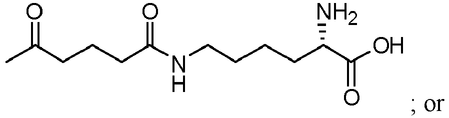 |
| (3) | 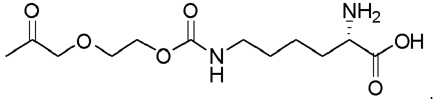 |

6. Use of the unnatural amino acid according to any one of claims 1-5 in preparation of a recombinant protein or a recombinant protein conjugate; preferably, the recombinant protein is a recombinant human growth hormone; or, preferably, the recombinant protein conjugate is a recombinant human growth hormone-polyethylene glycol conjugate.

7. A recombinant protein, wherein at least one site in the amino acid sequence of the recombinant protein is the unnatural amino acid according to any one of claims 1-5.

8. A recombinant protein conjugate, wherein the recombinant protein conjugate is formed by forming an oxime bond with the terminal carbonyl of the unnatural amino acid in the recombinant protein according to claim 7 and a coupling moiety containing an "NH₂-O-" terminal group.

9. The recombinant protein conjugate according to claim 8, wherein the recombinant protein is a recombinant human growth hormone, the coupling moiety containing the "NH₂-O-" terminal group is polyethylene glycol containing an "NH₂-O-" terminal group, and is preferably polyethylene glycol with a molecular weight of 10 KD to 100 KD; more preferably, an amino acid sequence of the recombinant human growth hormone is as shown in SEQ ID NO: 1; and further preferably, the corresponding 107^{th} site in the amino acid sequence of SEQ ID NO: 1 contains the unnatural amino acid according to any one of claims 1-5.

10. Use of the recombinant protein conjugate according to claim 9 in preparation of a drug for treating growth and development disorders caused by endogenous growth hormone secretion insufficiency, growth and development disorders caused by Turner syndrome or adult growth hormone deficiency.
